# EUROPÄISCHE PATENTSCHRIFT

(11) **EP 0 915 165 B1**
(45) Veröffentlichungstag und Bekanntmachung des Hinweises auf die Patenterteilung: **19.02.2003**
(21) Anmeldenummer: 98120625.3
(22) Anmeldetag: 02.11.1998
(51) Int. Cl.: C12N 15/86, C12N 5/10, C12N 7/01

(54) **Verfahren zur helfervirusfreien Verpackung einer Genvektor-DNA**
Method for the helper virus-free packaging of a DNA-vector
Méthode d'encapsidation de vecteur d'ADN recombinant dépourvu de virus auxiliaire

(30) Priorität: 05.11.1997 DE 19748895; 27.03.1998 DE 19813775
(43) Veröffentlichungstag der Anmeldung: 12.05.1999
(73) Patentinhaber: GSF-Forschungszentrum für Umwelt und Gesundheit GmbH, 85764 Oberschleissheim (DE)
(72) Erfinder: Delecluse, Henri-Jacques, 80335 München (DE); Pich, Dagmar, 80637 München (DE); Hammerschmidt, Wolfgang, 80686 München (DE)
(74) Vertreter: Reinhard - Skuhra - Weise & Partner

(56) Entgegenhaltungen:
- EP-A- 0 694 613
- WO-A-96/29421
- WO-A-97/05263
- NALDINI L. ET AL.: "IN VIVO GENE DELIVERY AND STABLE TRANSDUCTION OF NONDIVIDING CELLS BY A LENTIVIRAL VECTOR" SCIENCE, Bd. 272, Nr. 5259, 12. April 1996, Seiten 263-267, XP000583652
- O'CONNOR M ET AL: "CONSTRUCTION OF LARGE DNA SEGMENTS IN ESCHERICHIA COLI" SCIENCE, Bd. 244, Nr. 4910, 16. Juni 1989, Seiten 1307-1312, XP000051939
- KEMPKES B. ET AL.: " Immortalization of human primary B lymphocytes in vitro with DNA." PROC. NATL. ACAD. SCI. USA, Bd. 92, Nr. 13, 1995, Seiten 5875-58789, XP002094030
- KEMPKES B. ET AL.: "Immortalization of human B lymphocytes by a plasmid containing 71 kilobas pairs of Epstein-Barr virus DNA." JOURNAL OF VIROLOGY, Bd. 69, Nr. 1, 1995, Seiten 231-238, XP002094031
- LEIB D. A. AND OLIVO P. D.: "Gene delivery to neurons: Is herpes simplex virus the right tool for the job?" BIOESSAYS, Bd. 8, Nr. 15, 1993, Seite 547 547 XP002079297

## Beschreibung

### Stand der Technik

Die vorliegende Erfindung betrifft ein Verfahren zur helfervirusfreien Verpackung von Genvektor-DNA in die Viruspartikel eines Herpes-Virus sowie Eukaryonten-Helferzellen zur helfervirusfreien Verpackung von Genvektor-DNA in die Viruspartikel eines Herpes-Helfervirus, wobei ein Herpes-Virus mit einem Genom ≥ 100 kbp eingesetzt wird.

Epstein-Barr Virus (EBV) ist eines von acht bekannten Herpesviren des Menschen. Die DNA Sequenz des B95.8 Isolats von EBV ist bestimmt (Baer et al., 1984), und detaillierte wissenschaftliche Erkenntnisse sind vor allem über DNA-Elemente erarbeitet worden, die an den beiden Phasen des EBV maßgeblich beteiligt sind. In der sogenannten 'latenten Phase' geht das Virus eine stabile Wirtszellbeziehung ein, in der die Vitalität der Zelle nicht gestört wird, das virale DNA Genom aber als extrachromosomales Plasmid in den Wirtszellen repliziert und an die Tochterzellen weitergegeben wird. Die latente Phase kann mit der Transformation bzw. Immortalisation der latent infizierten Zellen einhergehen. Der plasmidale Replikationsursprung oriP ist das DNA-Element, das für die Aufrechterhaltung und Replikations des EBV-Genoms in der latenten Phase essentiell ist (Yates et al., 1985). Dieses DNA-Element ist auch in rekombinanten Plasmiden aktiv und hat als solches verschiedene Anwendungen erfahren.

In der sogenannten 'lytischen oder produktiven Phase' wird Virus freigesetzt, was die Expression von fast allen viralen Proteinen erfordert, die zur Genregulierung bzw. der Produktion von viralen Strukturkomponenten nötig sind. Die lytische Phase setzt aber auch zwei weitere DNA-Elemente des Virus voraus: den lytischen Replikationsursprung, oriLyt, der verantwortlich ist für die Amplifikation viraler DNA (Hammerschmidt and Sugden, 1988), die Terminal Repeats, TR, die Verpackungssignale darstellen, die für die Enkapsidierung der amplifizierten EBV DNA unabdingbar nötig sind (Hammerschmidt and Sugden, 1989; Zimmermann and Hammerschmidt, 1995).

Weitreichendes Interesse besteht an der genetischen Analyse der EBV Funktionen, an der Konstruktion von rekombinanten EBV Genomen und an EBV Genvektoren. Das Interesse an Genvektoren ist besonders groß, da zusätzliche, therapeutisch relevante Gene in geeignete Empfängerzellen eingebracht werden können. Allerdings muß dabei ausgeschlossen werden, daß unerwünschte Gene des EBV in die Zielzelle mitübertragen werden. Dieses Problem muß gelöst sein, um den Anforderungen an die Sicherheit dieser neuen Therapieform zu genügen und herpesvirale Genvektoren in der Gentherapie am Menschen einsetzen zu können.

Die Strategie bei der Produktion von Genvektoren zielt darauf ab, nur die Genvektoren selbst mit einer viralen Hülle zu versehen, die Freisetzung von sogenannten Helferviren aber zu unterbinden. Das wird erreicht, wenn cis-aktive DNA Elemente des Helfervirusgenoms entfernt oder mutiert werden, die bei der Produktion des Helfervirus selbst benötigt werden. Bisher war es aber unmöglich, die Teile des EBV Genoms, die für die Reifung von Viruspartikeln unabdingbar sind, genetisch zu manipulieren. Damit konnten die Funktionen des EBV oder anderer Herpesviren nicht ausgeschaltet werden. die z.B. für die Verpackung des viralen Genoms absolut notwendig sind. Konventionell wurden bislang Zellinien benutzt, die mit Herpesviren gezielt infiziert wurden, um alle Funktionen des Virus für die Amplifikation des Genvektors zu nutzen. Nicht vermeidbar war die gleichzeitige Vermehrung des infizierenden Virus und die Verpackung seines Genoms in eine Virushülle. In ähnlicher Weise wurden von uns auch Zellinien in der Vergangenheit benutzt, die latent mit EBV infiziert sind. Auch hier werden für die Verpackung von geeigneten Genvektoren lytische Funktionen des Virus benötigt, so daß ebenfalls eine Vermehrung des Helfervirusgenoms und die darauffolgende Verpackung nicht vermieden werden konnte (Hammerschmidt and Sugden, 1989; Kempkes et al., 1995b).

### Nachteile des Stands der Technik

Virale Genvektoren sind für die Gentherapie ein wichtiges Instrument. Allerdings muß gewährleistet sein, daß bei ihrer Herstellung und therapeutischer Anwendung die Sicherheit besteht, daß viralen Genvektoren hergestellt werden, die kein Virus enthalten. Da für die Produktion von viralen Genvektoren bestimmte virale Genfunktionen absolut notwendig sind, kann auf den Einsatz von sogenannten Helferviren oder ihrer funktionellen Abschnitte nicht verzichtet werden. Dies trifft besonders auf komplexe Virussysteme zu, die auf viele, viral kodierte Funktionen angewiesen sind. So kodiert z. B. EBV für mehr als 80 Gene, von denen ca. 50 für die Virussynthese unverzichtbar sind. Da diese Gene in ihrer Gesamtheit auf dem viralen Genom in einem einzigen DNA Molekül vorliegen, müssen dem Genom bestimmte Eigenschaften fehlen, so daß es nicht ebenfalls in virale Partikel verpackt und zusammen mit dem Genvektor freigesetzt wird. Eine Möglichkeit, diese Freisetzung des Helfervirus zu verhindern besteht darin, die cis-aktiven Genomabschnitte zu deletieren, die für die Verpackung des Helfervirusgenoms selbst essentiell sind. Diese Genomabschnitte werden bei EBV als TR-Sequenzen (TR=terminal repeats) bezeichnet, bei anderen Herpesviren spricht man von pac-Sequenzen (pac=packaging). Alternativ können auch andere Abschnitte deletiert oder in ihrer Funktion aufgehoben werden, die für die Replikation des Helfervirusgenomes in cis unabdingbar sind. Bei EBV kommt dafür der lytische Replikationsorigin, oriLyt, in Frage, dessen funktionelles Äquivalent bei anderen Herpesviren als oriS oder oriL bezeichnet wird.

Die Deletion dieser Abschnitte macht die Freisetzung von Helfervirus unmöglich, allerdings geht dabei auch die Möglichkeit verloren, das Helfervirus selbst zu erstellen oder gar via Infektion in die Zellen einzuführen, in denen der virale Genvektor verpackt werden soll. Da sich diese Eigenschaften ausschließen, gelang es bisher nicht, Zellinien zu erstellen, die herpesvirale Genvektoren ohne die Freisetzung von Helferviren zu produzieren. Gleiches gilt für Genvektoren, die auf anderen viralen Systemen aufbauen, die Virusgenome mit einer Größe von über 100 kb aufweisen. Dieser Nachteil kann auch nicht durch eine biochemische Trennung von Helfervirus und Genvektor behoben werden, da beide Partikel sich nur in Bezug auf ihre genetische Komposition und nicht in Bezug auf ihre äußere oder physikalische Beschaffenheit unterscheiden. Unsere Erfindung erlaubt es erstmals. diesen Nachteil zu beseitigen.

Es ist eine Aufgabe der vorliegenden Erfindung, ein Verfahren zur Verpackung einer Genvektor-DNA in die Viruspartikel eines Herpes-Virus bereit zu stellen, das zum einen die Proteine zur Herstellung der Viruspartikel eines Herpes-Virus mit einem Genom ≥ 100 kbp einsetzt und zum anderen die Verpackung der Helfervirus-DNA in die Viruspartikel vermeidet. Diese Aufgabe wird durch ein Verfahren mit den nachfolgenden Schritten gelöst:
(a) Einführen einer Herpes-Helfervirus-Vektor-DNA auf einem oder zwei Molekülen, mit zumindest den nachfolgenden Merkmalen:
   (α) einer Gesamtgröße ≥ 100 kbp;
   (β) mit zumindest einer Mutation, die so ausgelegt ist, daß eine oder mehrere der cis-aktiven Signalsequenzen zur Verpackung der Herpes-Helfervirus-Vektor-DNA nicht funktionell sind;
   (γ) mit der Information zur Herstellung der Herpes-Virus-Partikel des Herpes-Helfervirus, die kein Helfervirusgenom enthalten,
   (δ) mit der Information für ein in eukaryonten Zellen selektierbares Markergen;
   in eine Eukaryontenzelle;
(b) Etablieren einer Zelllinie, die die Herpes-Helfervirus-Vektor-DNA in stabiler Form enthält;
(c) Einführen einer zu verpackenden Genvektor-DNA mit zumindest
   (α) einer cis-aktiven Signalsequenz zur Verpackung der Genvektor-DNA in ein Viruspartikel des Herpes-Helfervirus;
   (β) einem Gen von Interesse
   in die Eukaryontenzelle.
(d) Induktion der lytischen Phase des Herpes-Helfervirus und Produktion der für die Verpackung wichtigen Proteine des Herpes-Helfervirus;
(e) Verpackung der Genvektor-DNA in die Viruspartikel des Herpes-Helfervirus; wahlweise
(f) Freisetzen von die Genvektor-DNA enthaltenden Viruspartikeln; und/oder wahlweise
(g) Aufreinigen der Viruspartikel.

Erfindungsgemäß kann die Herpes-Helfervirus-Vektor-DNA nicht nur auf einem Molekül, sondern auch auf zwei Molekülen vorliegen, wobei die Gesamtgröße der Moleküle ≥ 100 kbp beträgt.

Die Herpes-Helfervirus-Vektor-DNA ist so mutiert, daß eine oder mehrere der cis-aktiven Signalsequenzen zur Verpackung der DNA nicht funktionell sind, d.h. die Helfervirus-DNA zwar die Informationen für die Herstellung der Viruspartikel zur Verfügung stellen kann, nicht jedoch selbst in die Viruspartikel verpackt werden kann.

Die zu verpackende Genvektor-DNA enthält diejenigen cis-aktiven Signalsequenzen, die zu ihrer Verpackung in ein Viruspartikel des Helfervirus benötigt werden. Weiterhin enthält die zu verpackende DNA ein Gen von Interesse. In weiteren Ausgestaltungsformen der Erfindung kann auch zumindest ein Prokaryonten selektierbares Markergen vorhanden sein.

Nach Induktion der lytischen Phase des Herpes-Helfervirus, nachfolgend kurz "Helfervirus" gennant, werden die für eine Verpackung wichtigen Proteine des Helfervirus gebildet, wobei in einem anschließenden Schritt die Genvektor-DNA in die gebildeteten Viruspartikel des Helfervirus verpackt wird. Da der Herpes-Helfervirus-Vektor-DNA die Signalsequenzen zur Verpackung fehlen, wird Helfervirus-DNA nicht in die Viruspartikel verpackt.

Die Eukaryonten-Helferzelle, die die Viruspartikel des Helfervirus mit der verpackten Genvektor-DNA enthält, kann in der vorliegenden Form verwendet werden, wobei aber auch die Viruspartikel freigesetzt werden können und wahlweise aufgereinigt, isoliert und/oder konzentriert werden können.

In einer bevorzugten Ausgestaltungsform der Erfindung enthält die Herpes-Helfervirus-Vektor-DNA EBV-DNA. In der Helfervirus-DNA können, im Falle von EBV als Helfervirus, als cis-aktive Signalsequenz die terminalen Repeatsequenzen des EBV, der lytische Replikationsursprung des EBV, der plasmidale Replikationsursprung oriP des EBV oder das EBNA1-Gen des EBV so mutiert sein, daß sie ihre Funktion als cis-aktive Signalsequenz zur Verpackung nicht mehr wahrnehmen können. Umgekehrt können diese Signalsequenzen in der zu verpackenden Genvektor-DNA als Verpackungssignale in funktioneller Form eingesetzt werden.

Markergene, die in prokaryonten Zellen und/oder eukaryonten Zellen selektierbar sind, sind an sich bekannt. Beispiele hierfür sind Antibiotikumresistenzgene oder Gene, die für ein durch Fluoreszenz nachweisbares Protein kodieren.

Als Eukaryontenzelle kann jede Zelle eingesetzt werden, die mit der Herpes-Helfervirus-Vektor-DNA kompatibel ist.

In einer bevorzugten Ausgestaltungsform der Erfindung wird in die Eukaryontenzelle ein Gen zur Beeinflussung des Zelltropismus des Helfervirus eingeführt, wobei dieses Gen entweder auf einem Plasmid liegt, das von der Genvektor-DNA verschieden ist oder auf der Genvektor-DNA angeordnet ist. Dieses Gen kodiert für ein Protein, das in das Viruspartikel integriert wird, so daß dessen Zelltropismus verändert wird. Selbstverständlich ist es auch möglich, mehrere Gene zu verwenden, die für mehrere Proteine kodieren, um den Zelltropismus zu verändern, falls dies gewünscht oder benötigt wird.

In einer weiteren Ausführungsform der Erfindung werden Eurkaryonten-Helferzellen zur helfervirusfreien Verpackung von Genvektor-DNA in die Viruspartikel eines Herpes-Helfervirus bereitgestellt, wobei die Helferzelle zumindest enthält:
(a) eine Herpes-Helfervirus-Vektor-DNA ≥ 100 kbp, auf einem oder zwei Molekülen, mit einer Mutation, die so ausgelegt ist, daß eine oder mehrere der cis-aktiven Signalsequenzen zur Verpackung nicht-funktionell sind, und mit der Information zur Herstellung der DNA-Viruspartikel, die kein Helfervirus-Genom enthalten; und mit der Information für ein Eukaryontenzellen selektierbares Markergen;
(b) eine Genvektor-DNA mit zumindest
   (α) einer cis-aktiven Signalsequenz zur Verpackung der Genvektor-DNA in Viruspartikel des Helfervirus;
   (β) einem Gen von Interesse.

In einer weiteren Ausgestaltungsform der Erfindung enthält die Genvektor-DNA weiterhin eine oder mehrere Markergene, die in Prokaryonten selektionierbar sind.

Selbstverständlich kann die Eukaryonten-Helferzelle noch weitere DNA-Abschnitte, beispielsweise in Form von Plasmiden, enthalten. Die in der Eukaryonten-Helferzelle vorliegende Herpes-Helfervirus-Vektor-DNA und die Genvektor-DNA können wie vorstehend beschrieben ausgestaltet sein.

Die Eukaryonten-Helferzelle der vorliegenden Erfindung kann als Herpes-Helfervirus-Vektor-DNA bevorzugt eine EBV-DNA oder zumindest Teile hiervon, enthalten. Sie ist beispielsweise eine Humanzelle, wobei, wie bereits oben näher dargestellt, jede Zelle verwendbar ist, die mit dem Helfervirus kompatibel ist.

Bevorzugt wird erfindungsgemäß als EBV eingesetzt.

Die zum ersten Mal mögliche helfervirusfreie Verpackung einer Genvektor-DNA in die Hülle eines DNA-Virus mit einem Genom von ≥ 100 kbp wurde durch die Etablierung einer Technologie ermöglicht, die es erlaubt, das gesamte Genom von DNA-Viren ≥ 100 kbp in Prokaryonten zu klonieren und jeden beliebigen Abschnitt dieses großen DNA-Genoms in Prokaryonten, aber auch in Eukaryonten, zu verändern. Damit war es zum ersten Mal möglich, auch solche Abschnitte von DNA-Viren ≥ 100 kbp genetisch zu verändern, die für die Funktionen des Virus in Eukaryontenzellen absolut essentiell sind. Beispiele für solche Funktionen sind bei EBV die beiden "Origins" der DNA-Replikation von EBV, oriLyt und oriP, und Verpackungssignale der viralen DNA, die sogenannten Terminal Repeats.

Durch diese Technik konnten die Funktionen für die Verpackung und/oder die DNA-Replikation des Helfervirus-Genoms gezielt ausgeschaltet werden. Ausgangspunkt hierzu ist die Klonierung des Genoms von Herpes-Viren ≥ 100 kbp, beispielsweise von EBV, in eine Prokaryontenzelle, beispielsweise E. coli, in Form eines rekombinanten Plasmids. Dieser Meilenstein war die Voraussetzung, daß über herkömmliche rekombinante DNA-Technologien die Modifikation aller Abschnitte von DNA-Viren ≥ 10.0 kbp in Prokaryonten ermöglicht wird. Diese Modifikation schließt auch diejenigen Genomabschnitte von DNA-Viren ein, die für die Replikation des viralen Genoms und seine Verpackung in eukaryonte Zellen essentiell sind. Die Deletion oder der Funktionsverlust dieser Genomabschnitte in der Prokaryontenzelle hat keine Konsequenzen in der Prokaryontenzelle, da beispielsweise die EBV-Abschnitte nur das - sehr große - Insert in einem E. coli.Replikon darstellen, die für die Replikation und Aufrechterhaltung des Replikons in E. coli ohne Bewandnis sind. Das so in einer Prokaryontenzelle modifizierte virale Genom läßt sind in Form von Plasmid-DNA isolieren, die dann über herkömmliche DNA-Transfektionstechniken in geeignete Eukaryontenzellen eingebracht wird. Dieses Herstellungsverfahren erlaubt die Etablierung von Helfervirusgenomen, die virale Genvektoren in eine Virushülle verpacken können, selbst aber nicht mehr freigesetzt werden. Diese Verfahren wird nachfolgend näher beschrieben. Die Beschreibung erfolgt insbesondere auf der Grundlage der Klonierung des gesamten EBV-Genoms als Plasmid in eine E. coli-Zelle. Die Klonierung von EBV und die Herstellung einer verpackungsfreien Helferzellinie, die eine EBV-Hülle bereit stellt, ist jedoch lediglich als beispielhalfte Ausgestaltungsform der Erfindung zu verstehen. Die Erfindung kann allgemein auch auf andere Herpes-Viren übertragen werden, die ein Genom ≥ 100 kbp besitzen.

Die Erfindung wird nachfolgend anhand von Ausführungsbeispielen und anhand von Abbildungen näher beschrieben. Die Erfindung wird am Beispiel einer EBV-Helfervirus-Vektor-DNA beschrieben. Dies ist jedoch nur als beispielhafte Ausgestaltung der Erfindung zu verstehen. Erfindungsgemäß können auch andere Herpes-Helferviren, eine andere Genvektor-DNA usw. eingesetzt werden. Die Erfindung ist nicht auf die nachfolgenden Beispiele beschränkt.

Ausgangsmaterial für die Erstellung eines Helfervirusgenoms, das für eine Verpackungszellinie für herpesvirale Genvektoren geeignet ist, waren Plasmide aus E. coli, die das gesamte EBV Genom des EBV Stammes B95.8 tragen. Die Herstellung solcher EBV/F-Faktor Plasmide wird nachfolgend beschrieben.

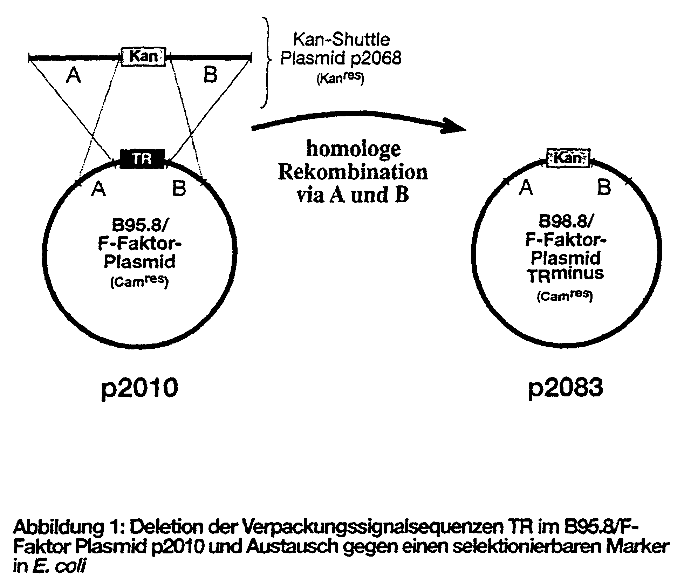

Um die für die Verpackung des Virus wichtigen cis-aktiven Abschnitte zu entfernen, wurden die Terminal Repeats in dem EBV/F-Faktor Plasmid in E. coli deletiert, bzw. der prokaryonte Selektionsmarker Kanamycinphosphotransferase an diese Stelle inseriert. Im einzelnen ist dieses Verfahren in Abbildung 1 dargestellt. Alternativ kann auch durch andere Verfahren die Deletion dieses cis-aktiven Elements erreicht werden. Die Aufgabe kann auch dadurch gelöst werden, daß die sogenannten lytischen Replikationsorigins von EBV funktionslos gemacht werden, so daß kein replikatives Intermediat des viralen Genoms entsteht, das für seine Verpackung Voraussetzung ist.

Das so veränderte, rekombinante EBV/F-Faktor Plasmid kann aus E. coli isoliert, und die DNA Moleküle können über herkömmliche Methoden präpariert werden. Diese DNA Moleküle werden nun über DNA Transfertechniken in eukaryonte Zellen stabil oder transient eingeführt. Die rekombinanten EBV Genome replizieren extrachromosomal in diesen Zellen wie in latent EBV infizierten Zellinien. In diesen Zellinien kann die lytische Phase von EBV induziert werden, was zur Expression aller für die Virussynthese wichtigen Proteine führt. Prinizpiell stehen diese Proteine in der Zelle in trans auch für die DNA Replikation und Verpackung von anderen rekombinanten Plasmiden, sogenannten mini-EBV-Plasmiden oder Genvektoren, in Viruspartikel zur Verfügung (Hammerschmidt and Sugden, 1988: Hammerschmidt and Sugden. 1989).

Da dem modifizierten EBV/F-Faktor Plasmid die cis-aktiven DNA-Elemente fehlen. die für seine eigene Verpackung essentiell sind, kommt es zur Synthese von leeren. defekten Epstein-Barr Viruspartikeln, die keine genetische Information tragen, die aber in den Zellkulturüberstand freigesetzt werden können. Damit ist eine Zellinie entstanden, die alle Funktionen für die Verpackung von virale Genvektoren bereitstellt, ohne daß das EBV/F-Faktor Plasmid als Helfervirusgenom verpackt werden kann. Solche Zellinien werden auch als helfervirusfreie Verpackungszellinien bezeichnet.

Virale Genvektoren sind rekombinante Plasmide, die i. d. R. mit herkömmlichen molekularbiologischen Klonierungsverfahren hergestellt werden. Für die meisten Genvektoren sind mindestens zwei virale Genomabschnitte nötig, die in cis auf dem rekombinanten Plasmid vorliegen müssen. Bei diesen beiden Abschnitten handelt es sich zum einen um die Verpackungssignale, die für die Enkapsidierung der Genvektor-DNA in virale Partikel essentiell sind. Zum anderen muß der Replikationsorigin auf dem DNA-Molekül vorliegen. Die Amplifikation der Genvektor-DNA, die über den Replikationsorigin erfolgt, ist nicht nur für die DNA Replikation und die Erhöhung der Kopienzahl der Genvektor-DNA notwendig, sondern vor allem für die Synthese von replikativen DNA-Intermediaten. Bei Herpesviren sind diese DNA-Intermediate konkatemere DNA-Moleküle, die aus vielen tandemartig angeordneten Kopien des DNA-Ausgangsmoleküls bestehen. Man ist der Auffassung, daß diese replikativen DNA-Intermediate die Voraussetzung für die Enkapsidierung von Nukleinsäuremoleküle in Viruspartikel sind. Für den Schritt der Enkapsidierung sind aber auch die Verpackungssignale entscheidend, wie bereits oben ausgeführt.

Virale Genvektorplasmide, die im Fall von EBV die Verpackungssignale TR und den lytischen Replikationsorigin oriLyt neben den für die Propagierung in E. coli wichtigen prokaryonten Plasmidanteile tragen, sind prinzipiell für die Verpackung in eine EB-Virushülle geeignet. Solche Genvektorplasmide werden in transienter oder stabiler Form in die helfervirusfreie Verpackungszellinie eingebracht, und die lytische Phase von EBV wird induziert. Über die vom EBV/F-Faktor Plasmid als Helfervirusgenom bereitgestellten viralen Proteine kommt es zur DNA-Replikation des viralen Genvektorplasmids, zur Verpackung der Genvektor-DNA in virale Partikel und zu deren Freisetzung.

### Ausführungsbeispiele

### 1. Deletion der TR-Abschnitte auf einem EBV/F-Faktor Plasmid als Helfervirusgenom

Ein EBV/F-Faktor Plasmid. mit p2010 bezeichnet, wurde in E. coli über beschriebene Techniken so modifiert, daß die Verpackungssignale TR deletiert und das prokaryonte Markergen Kanamycinphosphotransferase an diese Stelle eingefügt ist. Wie in Abbildung 1 schematisch gezeigt, wurde diese Veränderung im EBV/Faktor Plasmid mit der als selektive Integration beschriebenen Technik durchgeführt. Details zur Ausführung sind in Abbildung 1 aufgenommen, bzw. im Abbildungstext beschrieben. Das modifizierte EBV/F-Faktor Plasmid wird mit p2083 bezeichnet.

Alternative Methoden zur genetischen Modifikation von F-Faktor klonierter genomischer EBV DNA bestehen und werden nachfolgend beschrieben. Antibiotikaresistenzmarker und Sequenzen, die für die Rekombination eingesetzt werden, sind beliebig wählbar.

Das TR-minus EBV/F-Faktor Plasmid p2083 wurde aus E. coli isoliert und durch herkömmliche DNA-Transfektionstechniken in die humane Zellinie 293 transferiert. Das TR-minus EBV/F-Faktor Plasmid trägt das Gen für den eukaryonten Selektionsmarker Hygromycinphosphotransferase. Die transfizierten 293 Zellen wurden mit geeigneten Konzentrationen an Hygromycin selektioniert. Einzelne Zellklone wurden auf ihre Eigenschaften funktionell untersucht.

### 2. Charakterisierung der helfervirusfreien Verpackungszellinie

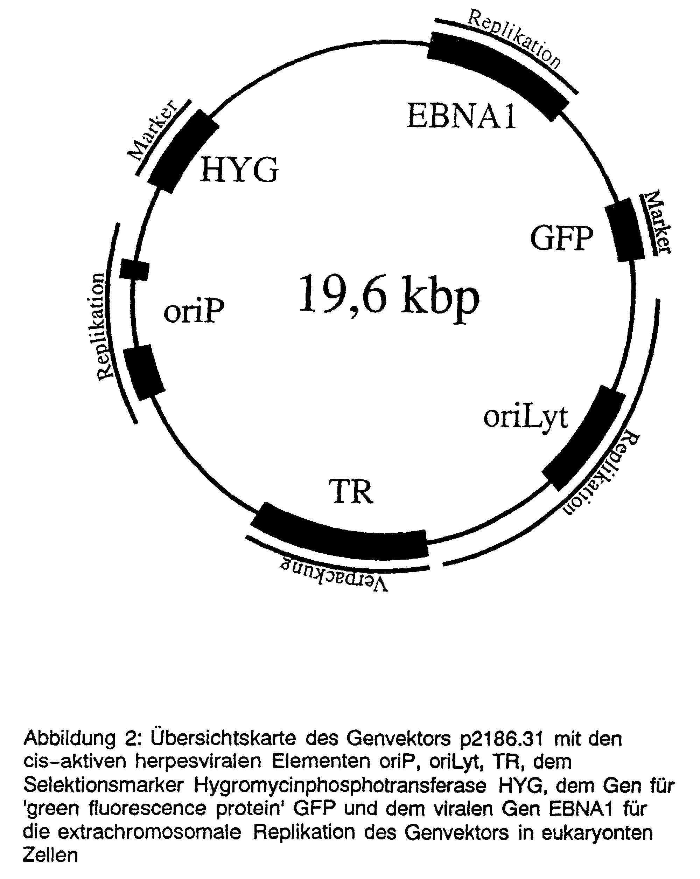

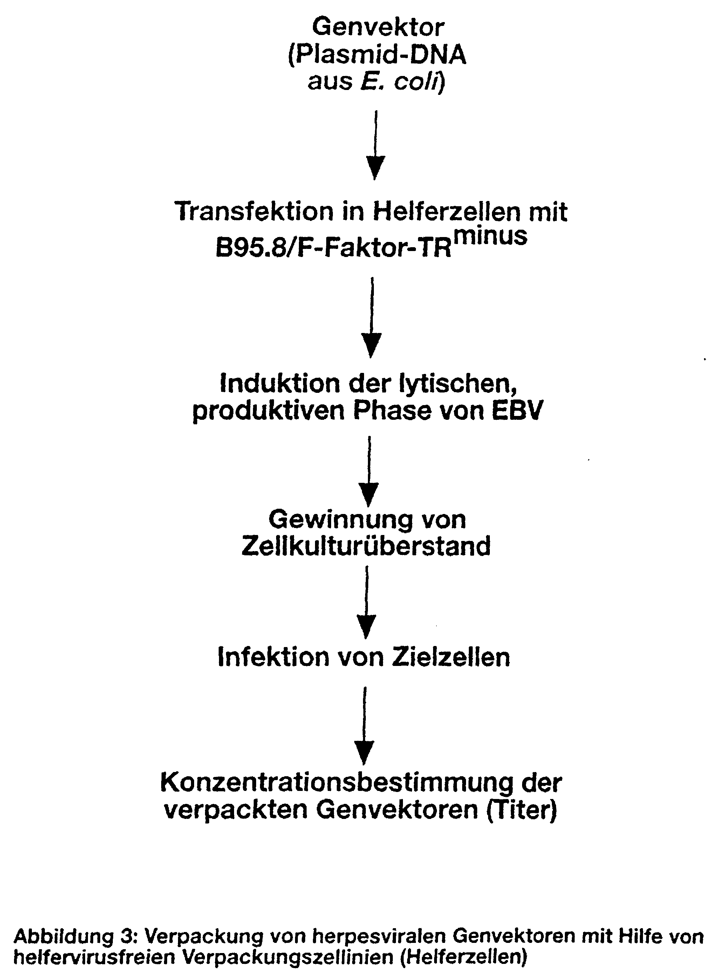

Ein viraler Genvektor p2186.31 wurde als Plasmid mit herkömmlichen DNA-Klonierungstechniken konstruiert, wie in Abbildung 2 gezeigt. Dieses Plasmid dient als Reporterplasmid zur Charakterisierung der Verpackungsfunktionen von 293 Zellen, die das TR-minus EBV/F-Faktor Plasmid tragen. Der virale Genvektor trägt neben den Verpackungssequenzen TR und dem lytischen Replikationsorigin oriLyt auch weitere virale Sequenzen, die für die extrachromosomale Replikation des Genvektors in der Rezipientenzelle essentiell sind. Es handelt sich dabei um den plasmidalen Replikationsorigin oriP und das Gen EBNA1 (Abbildung 2). Darüber hinaus trägt der Genvektor das Gen für das 'green fluorescence protein' und das Gen für Resistenz gegenüber Hygromycin. Der virale Genvektor wird wie in Abbildung 3 gezeigt transient in diese Zellen eingebracht und gleichzeitig der lytische Zyklus von EBV mit einem Expressionsplasmid für das virale Gen BZLF1 induziert, wie beschrieben (Hammerschmidt and Sugden, 1989). Der Überstand dieser Zellen enthält den in eine EBV-Hülle verpackten Genvektor. Die Analyse und Konzentrationsbestimmung des Genvektors erfolgt durch Infektion geeigneter Zellen, die den Rezeptor für EBV tragen, z. B. Raji-Zellen. Die erfolgreiche Infektion von Raji-Zellen mit dem Genvektor p2186.31 kann einfach über die Fluoreszenz des 'green fluorescence proteins' bestimmt werden, da mit dem Genvektor infizierte Zellen unter UV-Beleuchtung im Mikroskop grün erscheinen.

Darüber hinaus kann der erfolgreiche Transfer der Genvektor DNA p2186.31 durch Selektion der infizierten Raji-Zellen mit Hygromycin bestimmt werden. Die anschließende DNA-Analyse der hygromycinresistenten Raji-Zellen mit der Southern Blot Technik und geeigneten radioaktiven DNA-Sonden bestätigt die Anwesenheit intakter Genvektor DNA und die Abwesenheit von TR-minus EBV/F-Faktor Plasmid oder Teilen davon.

Alternativ kann als viraler Genvektor auch ein Plasmid eingesetzt werden, dem die Anteile oriP und EBNA1 fehlen oder das an Stelle des Gens für das 'green fluosrescence protein' GFP ein oder mehrere therapeutisch interessante Gene trägt. Auch kann gegebenenfalls auf den Selektionsmarker Hygromycinphosphotransferase verzichtet werden oder ein anderes Gen an seine Stelle treten.

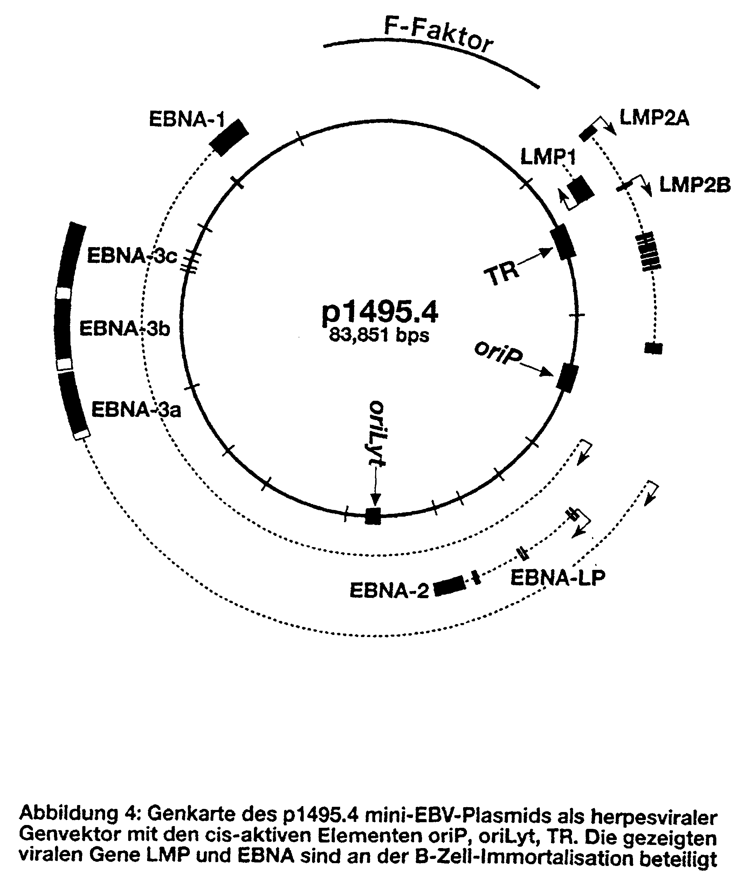

Alternativ kann als viraler Genvektor ein mini-EBV-Plasmid eingesetzt werden, das neben den viralen Sequenzen für oriLyt, TR, EBNA1 und oriP noch weitere Gene besitzt, die für die Immortalisation von humanen B-Zellen benötigt werden. Solche mini-EBV-Plasmide sind in ihren Eigenschaften von uns beschrieben, und ein Beispiel ist in Abbildung 4 gezeigt (Hammerschmidt and Sugden, 1989; Kempkes et al., 1995a; Kempkes et al., 1995b). Auch dieses mini-EBV-Plasmid kann transient oder stabil in die Verpackunszellinien eingebracht werden, der lytische Zyklus von EBV induziert werden und der Überstand der Zellen auf verpackte mini-EBV-Plasmide untersucht werden. Mit dem Überstand werden humane primäre B-Lymphozyten infiziert, die dann als immortalisierte, permant proliferierende B-Zellinien auswachsen. Alle B-Zellinien enthalten eine oder mehrere Kopien des mini-EBV-Plasmids aber keine TR-minus EBV/F-Faktor Plasmide oder Teile davon.

### 3. Genvektoren für die Transduktion in bestimmte Zellen.

Genvektoren übertragen Gene in solche Zellen, die von den korrespondierenden Helferviren normalerweise infiziert werden. Helferzellinien eröffnen die Möglichkeit, diesen sogenannten Zelltropismus zu beeinflussen. Glykoproteine von Herpesviren, die für die Infektion bestimmter Zellen (bei EBV natürlicherweise B-Zellen und einige Epithelzellen) verantwortlich sind, können durch Mutation der F-Faktor klonierten EBV Genome in E. coli verändert oder gegen andere ausgetauscht werden. Ein Beispiel für einen erweiterten Zelltropismus ist das Vesikulostomatitisvirus-Glykoprotein G, ein Beispiel für den extrem spezifischen Zelltropismus für fast ausschließlich pluripotente hämatopoetische Stammzellen ist der Ligand für den Stem Cell Factor Receptor auf der Stammzelle, der membranständige Stem Cell Factor Ligand (mSCF-ligand). Dieser Vorgang wird als Pseudotyping bezeichnet und ist besonders für herpesvirale Vektoren attraktiv. Die für die Infektiosität wichtigen Glykoproteine sind bei Herpesviren in einer von der Kernmembran abgeleiteten Phospholipidmembran eingebettet. Diese Virushülle ist nicht wesentlich strukturiert und umschließt das streng geometrische Kapsid des Virions nur locker. Im Gegensatz zu unbehüllten Viren (z.B. Adenovirus, Adenoassoziiertes Virus, AAV) oder anderen behüllten Viren (z.B. Retroviren) ist die Hülle von Herpesviren und anderen großen DNA Viren flexibel und setzt keine bestimmte Struktur, Proteinkonfiguration oder -faltung voraus, die anderenfalls die Virusreifung und - assemblierung verhindern. Das ist die Voraussetzung, um die Genvektoren mit anderen Glykoproteine und damit neuen Eigenschaften in Bezug auf ihren Zelltropismus zu versehen.

Bei EBV werden zwei Glykoproteine bei der Infektion von Zellen als wichtig erachtet. Das Glykoprotein gp350/220 ist für die Adsorption der viralen Partikel an den zellulären Rezeptor entscheidend (Moore et al., 1987; Nemerow et al., 1989). Dieser Rezeptor wird auch als CD21 bezeichnet. Bei der Fusion der herpesviralen Hülle mit der Plasmamembran der Zelle ist das virale Glykoprotein gp85 beteiligt. Der zelluläre Gegenrezeptor scheint das MHC Klasse II Molekül zu sein. Für die Zelltargetierung ist vor allem gp350/220 entscheidend.

Zwei Verfahren werden von uns eingesetzt, um den Zelltropismus von herpesviralen Genvektoren zu verändern. Beide Verfahren beruhen auf der Expression von Glykoproteinen mit anderen Eigenschaften in der Helferzelle während der Verpackung von viralen Genvektoren. Die Glykoproteine müssen die Eigenschaften haben, in die Hülle dieser Genvektoren eingebaut zu werden, was über bestimmte Proteindomänen der Glykoproteine gewährleistet ist. Zum einen kann die Expression von Glykoproteinen mit anderem Zelltropismus transient durch Transfektion eines Expressionsplasmids erreicht werden, was gleichzeitig mit der Induktion des lytischen Zyklus in den Helferzellen vorgenommen werden kann. Zum anderen können Gene für Glykoproteine mit anderem Zelltropismus auch in das Helfervirusgenom oder das zelluläre Chromosom integriert werden, um während der Verpackung der Genvektoren zur Verfügung zu stehen. Beide Verfahren liefern gleichwertige Ergebnisse. Das funktionell analoge EBV-Glykoprotein gp350/220 auf dem Helfervirusgenom kann, aber muß nicht, deletiert sein.

### Vorteile gegenüber dem Stand der Technik

Das beschriebene Verfahren erlaubt die Herstellung von Genvektoren, die in eine herpesvirale Hülle verpackt sind. Der Vorteil dieses Verfahrens besteht darin, daß eine Helferzellinie eingesetzt wird, die keine Helferviren bei der Verpackung von Genvektoren freisetzt. Dieses Verfahren konnte bisher nicht mit Viren realisiert werden, deren Genome größer als die von Adenoviren (36 kbp) sind Mit Sicherheit kann man davon ausgehen, daß dieses Verfahren auf alle Herpes-Viren ≥ 100 kbp und abgeleitete Helferzellinien ausgedehnt werden kann. Die Herstellung solcher Helferzellinien setzt die Klonierung dieser Virusgenome in einen anderen Organismus, z. B. E. coli voraus, um die gezielte Veränderung viraler Abschnitte zu ermöglichen. Die so hergestellten viralen Genome weisen die Eigenschaft auf, alle viralen Genprodukte für die Amplifikation und Verpackung von viralen Genvektoren zu produzieren, aber kein Helfervirus freizusetzen. Diese Genvektoren können auch neue Eigenschaften aufweisen, so daß sie Zellart(en) infizieren, die von dem Helfervirus normalerweise nicht oder nur sehr ineffizient infiziert werden. Die Selektivität des Zelltropismus ist eine wichtige Voraussetzung zur Anwendung der Genvektoren in der Gentherapie. Es muß sicher gestellt sein, daß die über Vektoren vermittelte Transduktion von therapeutisch interessanten Genen ausschließlich in vorherbestimmte Zielzellen und nicht in beliebigen Zellen erfolgt.

Als denkbare Anwendungen hier einige Beispiele:
- Herpesvirale Genvektoren, die ein oder mehrere Gene an Stelle des Gens für das 'green fluorescence protein' tragen, sind für eine Anwendung in der Gentherapie beim Menschen interessant. Als therapeutische Gene kommen solche in Frage, die den Rezipientenzellen oder dem Gesamtorganismus neue Eigenschaften vermitteln. Diese Gene können zur Korrektur genetischer Erkrankungen dienen (z.B. ADA, Thalassämie, lysosomale Speichererkrankungen. Gerinnungsfaktor VIII, etc).
- Die Expression von bestimmten Antigenen kann dazu verwendet werden, um die mit Genvektoren transduzierten Zellen zur Immunisierung einzusetzen (z.B. als zelluläre Tumorvakzine gegen B- und T-Zellymphome).
- Genvektoren können ein oder mehrere Zytokine oder andere Signalmediatoren tragen, die bestimmte Eigenschaften haben (z.B. anti-inflammatorische Zytokine), die lokal bei chronischer Arthritis und anderen Erkrankungen sinnvoll eingesetzt werden können.

Wie eingangs bereits dargestellt ist eine unabdingbare Voraussetzung für die Ausführbarkeit der vorliegenden Erfindung die Möglichkeit, eine DNA-Helfervirus-Vektor-DNA bereitzustellen. Nachfolgend wird die Herstellung eines derartigen Helfervirus-Vektors, der ein Virus-Genom ≥ 100 kbp enthält, näher beschrieben.

Es war bisher nicht möglich, gezielt und mit hoher Effizienz jedes beliebige Gen von DNA-Viren >=100 kbp, beispielsweise des EBV, zu verändern oder zu deletieren. So sind die homologen Rekombinationsereignisse zwischen einem in E. coli klonierten Abschnitt von EBV und dem endogenen EBV, das in latent infizierten Zellinien vorhanden ist, ungenau, schwer zu kontrollieren und müssen über kotransfizierte Markerabschnitte oder selektionierbare Gene sichtbar gemacht werden. Beim derzeitigen Stand der Technik sind diese Ansätze nicht universell anwendbar, langsam, und erlauben nicht, Gene oder genomische Abschnitte von DNA Viren >= 100 kbp von EBV zu verändern. die z.B. für die Aufrechterhaltung der latenten Phase des EBV-Virus in den infizierten Zellen notwendig sind.

Es werden somit die Nachteile des Standes der Technik dadurch behoben, daß das gesamte Herpes-Virus-Genom von Viren mit einer Größe >= 100 kbp als funktionsfähige Einheit kloniert wird. Die Klonierung in z.B. E. coli als rekombinantes Molekül setzt voraus, daß es gelingt, einen sogenannten prokaryonten Genabschnitt in das intakte DNA-Virus-Genom zu integrieren. Die Integration eines solchen Abschnitts, der Replikationsfunktionen und einen selektionierbaren Marker für E. coli trägt, stellt sicher, daß so ein rekombinantes Molekül in der Größe von mehr als 100 kbp in z.B. E. coli transferierbar ist und dort stabil repliziert. Dieser Vorgang stellt zugleich sicher, daß über herkömmliche rekombinante DNA Technologien die Modifikation aller DNA-Virus-Abschnitte, z.B. in E. coli. möglich ist.

Nachfolgend wird das erfindungsgemäße Verfahren am Beispiel von EBV, beschrieben.

Das beschriebene Verfahren erlaubt die Herstellung von rekombinanten Herpesvirusgenomen. die in E. coli klonal vermehrt werden können. Mit Sicherheit kann man davon ausgehen. daß dieses Verfahren auf alle großen DNA Viren angewandt werden kann (z B. Poxviren, Iridioviren, andere Herpesviren, Baculoviren). Die Klonierung dieser Virusgenome in z.B. E coli erlaubt die einfache Modifikation durch gezielte Veränderungen viraler Gene, ihre Deletion oder das Hinzufügen von weiteren Genen, die von Interesse sind. Die so hergestellten viralen Genome weisen je nach genetischer Modifikation neue Eigenschaften auf, die z. B. die Expression von Fremdproteinen in den jeweiligen Zielzellen der Viren erlauben.

Die in z.B. E. coli klonierten viralen Genome stellen ein Produkt dar, das sich zur Herstellung von Viruspartikel einsetzen läßt, die z. B. für die Enkapsidierung von rekombinanten Plasmiden oder subgenomischen viralen Abschnitten geeignet sind.

Das Verfahren dient zur Herstellung von Herpes-Virus-Vektoren, die sowohl in eukaryontischen als auch prokaryontischen Zellen replizierbar sind und umfaßt zumindest die nachfolgenden Schritte:
a) Einbringen eines Herpes-Virus-Genoms ≥ 100 kbp in eine eukaryontische Zielzelle und Etablieren solcher Zellen, die das virale DNA-Genom zumindest in extrachromosomaler Form enthalten;
b) Einbringen eines DNA-Abschnitts in die eukaryontische Zielzelle, der zumindest, in funktioneller Anordnung, die Information für die Replikation des viralen Herpes-Genoms in prokaryontischen Zellen und zumindest ein in prokaryontischen Zellen selektierbares Markergen umfaßt, und der von DNA-Abschnitten (homologe Abschnitte) aus dem Herpes-Virus-Genom in einer eine Rekombination ermöglichenden Länge flankiert ist;
c) Integration der unter (b) bezeichneten Abschnitte in das Herpes-Virus-Genom durch Rekombination; wahlweise
d) Selektion auf diejenigen Zielzellen, die ein extrachromosomales, rekombinantes, virales Herpes-Genom enthalten; wahlweise
e) Aufreinigen und Isolieren des rekombinanten Herpes-Virus-Vektor-Genoms.

Dieses Verfahren unterscheidet sich von bisher bekannten Verfahren zur Herstellung von DNA-Virus-Vektoren insbesondere dadurch, daß zum ersten Mal die Herstellung derartiger Vektoren aus Herpes-Viren mit einer Größe >= 100 kbp ermöglicht wird. In bisherigen Verfahren wurden beispielsweise DNA-Viren wie Adeno-Viren mit einer Größe von ca. 40 kbp kloniert. Die Herstellung von Herpes-Virus-Vektoren mit einer Größe von >= 100 kbp, insbesondere >= 120 kbp. bevorzugt >= 150 kbp oder insbesondere bevorzugt >= 170 kbp war mit den aus dem Stand der Technik bekannten Verfahren nicht möglich.

Durch dieses Verfahren können rekombinante Herpes-Virus-Genome hergestellt werden, die sowohl in prokaryonten Zellen als auch eukaryonten Zellen vermehrbar sind.

Das Herpes Virus kann ein Alpha-Herpes-Virus, ein Beta-Herpes-Virus oder ein Gamma-Herpes-Virus sein. Beispiele für diese Viren sind: Alpha-Herpes-Virus: Herpes-Simplex-Virus (HSV), Varicella-Zoster-Virus (VZV); Beta-Herpes-Virus: Cytomegalie-Virus (CMV); Gamma-Herpes-Virus: Epstein-Barr-Virus (EBV)

Das Einbringen des Herpes-Virus in die eukaryontische Zielzelle kann durch an sich bekannte Methoden erfolgen; Beispiele hierfür sind Infektion, Transfektion oder Elektroporation. Gleiches gilt für das Einbringen des DNA-Abschnitts in die eukaryontische Zielzelle im Schritt (b).

Alternativ zum Verfahrensschritt (a), bei dem das Herpes-Virus in die eukaryontische Zielzelle eingeführt wird, können solche Zellen verwendet werden, die das Herpes-Virus bereits in extrachromosomaler Form enthalten.

Als Zielzelle ist jede Zelle zu verstehen, die Rezeptoren für das Virus besitzt und in denen das Virus replizieren kann.

Neben dem Herpes-Virus wird weiterhin ein DNA-Abschnitt in die eukaryontische Zielzelle eingebracht, der mindestens die Information für die Replikation des Herpes-Genoms in prokaryontischen Zellen und ein in prokaryontischen Zellen selektierbares Marker-Gen umfaßt. Diese Genabschnitte werden von DNA-Abschnitten flankiert, die eine solche Länge aufweisen, daß eine Rekombination mit dem Herpes-Virus ermöglicht wird. Die flankierenden DNA-Abschnitte weisen Homologien zum Herpes-Virus auf, so daß eine Rekombination, bevorzugt eine homologe Rekombination, ermöglicht wird. Die Länge der flankierenden DNA-Sequenzen liegt bevorzugt bei >= 300 bp, weiterhin bevorzugt bei >= 1 kbp und insbesondere bevorzugt bei >= 2 kbp.

Die Integration der im Schritt (b) bezeichneten Abschnitte in das Herpes-Virus-Genom erfolgt bevorzugt durch eine homologe Rekombination in der Zielzelle des Herpes-Virus. Es ist aber auch möglich, daß eine illegitime Rekombination zum gewünschten Ergebnis führt.

Normalerweise wird jedoch eine homologe Rekombination das rekombinierende Herpes-Virus-Genom ermöglichen.

Der DNA-Abschnitt im Schritt (b) kann vor dem Einbringen in die eukaryontische Zielzelle linearisiert werden. Die Enden werden so gegen einen Abbau durch endogene Nukleasen geschützt.

In einer bevorzugten Ausführungsform der Erfindung befinden sich die Information für die Replikation des Herpes-Genoms in Prokaryonten und die Information für das in prokaryontischen Zellen selektierbare Marker-Gen auf dem F-Plasmid von E. coli. In einer weiteren bevorzugten Ausführungsform der Erfindung enthält der DNA-Abschnitt im Schritt (b) zusätzlich zumindest ein in eukaryonten Zellen selektierbares Markergen. Als selektierbare Marker können beliebige, in prokaryonten Zellen bzw. in eukaryonten Zellen selektierbare Marker verwendet werden. Beispiele hierfür sind insbesondere Antibiotikaresistenzgene und Fluoresenzmarkergene.

F-Faktor abgeleitete Plasmide mit ca. 5 kpb enthalten in der Regel folgende Abschnitte und Gene des E. coli F-Faktorplasmids, das in der natürlich vorkommenden Form um die 100 kpb groß ist: für die Aufrechterhaltung der Kopienzahl um 1 bis 2 Kopien/E. coli Zelle sind die Gene parA und parB wesentlich, für die DNA-Replikation werden oriS und das Gen repE benötigt. Alle diese Elemente befinden sich auf dem F-Faktor-Plasmid. das z.B. für die Generation des EBV/F-Faktor-Konstrukts verwandt wurde.

In einer weiteren Ausführungsform befindet sich auf dem DNA-Abschnitt im Verfahrensschritt (b) zumindest ein Gen von Interesse, das beispielsweise für ein Protein der Blutgerinnungskaskade codiert, beispielsweise für das Faktor VIII-Gen. Beliebige, an sich bekannte Gene können auf dem DNA-Abschnitt lokalisiert sein und durch das erfindungsgemäße Verfahren in den Herpes-Virus-Shuttle-Vektor eingebracht werden. Hierdurch ist es möglich, das Gen von Interesse entweder in einer eukaryonten Zelle oder in einer prokaryonten Zelle zu exprimieren.

Durch dieses Verfahren wird die Klonierung vollständiger Herpes-Virus-Genome mit einer Größe >= 100 kbp ermöglicht. Ein besonders bevorzugtes Beispiel ist das EBV-Genom in einer Größe von ca. 170 kbp.

In einer Ausführungsform werden die homologen Abschnitte in(b) so gewählt, daß eine Mutation in das virale Genom eingeführt wird. Unter Mutation wird erfindungsgemäß eine Punktmutation, eine mehrere Nukleotide betreffende Mutation, eine Deletion, eine Addition oder ein Austausch von Nukleotiden verstanden. Die Addition von Nukleotiden umfaßt auch das Einführen von ein oder mehreren Genen, die beispielsweise für ein Gen von Interesse, zumeist ein therapeutisch wertvolles Gen, kodieren können.

In einer Ausführungsform des Verfahrens werden die flankierenden Abschnitte in Verfahrensschritt (b) so ausgewählt, daß die terminalen Repeats im herpesviralen Genom deletiert werden. Nach Rekombination mit dem Herpes-Virus-Genom entsteht ein verpackungsdefektes virales Genom, wodurch nach Induktion der lytischen Phase von EBV in den Zielzellen keine infektiösen Partikel freigesetzt werden, während gleichzeitig jedoch alle viralen Proteine und Funktionen zur Verpackung von DNA-Molekülen in trans zur Verfügung gestellt werden.

In einer weiteren Ausführungsform wird das erhaltene rekombinante Herpes-Virus-Vektor-Genom in weiteren Verfahrensschritten mutiert. Eine Definition, was erfindungsgemäß unter "Mutation" zu verstehen ist, wurde bereits weiter oben gegeben. Die Einführung der Mutationen kann sowohl in der oben beschriebenen Weise erfolgen, aber auch dadurch, daß das erhaltene Herpes-Virus-Vektor-Genom in eine Prokaryontenzelle eingeführt wird oder eine andere Eukaryontenzelle und in diesen Zellen, beispielsweise durch weitere Rekombinationsereignisse, die Mutationen eingeführt werden. In einer bevorzugten Ausführungsform der Erfindung wird eine Mutation auf einem oder mehreren der cis-aktiven Abschnitte des Herpes-Genoms eingeführt, wobei die Mutation die Verpackung des viralen Genoms verändert.

In einer erfindungsgemäß besonders bevorzugten Ausgestaltung werden die prokaryonten Anteile des DNA-Abschnitts im Schritts (b) so ausgewählt, daß sie zumindest teilweise zueinander homologe Sequenzen aufweisen, die eine solche Länge besitzen, daß eine homologe Rekombination ermöglicht wird, wodurch die prokaryonten Anteile des DNA-Abschnitts aus (b) eliminiert werden. Durch eine derartige homologe Rekombination werden die im erfindungsgemäßen Verfahren eingeführten prokaryonten Anteile in nachfolgenden Verfahrensschritten wieder eliminiert. Hierdurch können beispielsweise Antibiotikaresistenzen, die durch das Einbringen des DNA-Abschnitts in die eukaryontische Zielzelle eingeführt wurden und weitere prokaryonte Anteile wieder entfernt werden, so daß ein für die Gentherapie verwendbarer Herpes-Virus-Vektor zur Verfügung steht, der einen möglichst geringen Anteil prokaryonter Fremdsequenzen besitzt. Ein Beispiel hierfür wird in den Ausführungsbeispielen näher beschrieben.

Weiterhin wird ein Herpes-Virus-Vektor bereitgestellt, der, in funktioneller Anordnung, zumindest ein Herpes-Virus-Genom ≥ 100 kbp, zumindest ein in prokaryontischen Zellen selektierbares Markergen und die Information für die Replikation des Herpes-Genoms in prokaryontischen Zellen umfaßt.

Die beiliegenden Abbildungen zeigen:
- **Abb. 5:**: Klonierung genomischer B95.8 DNA in E. coli mit Hilfe eines F-Faktor Plasmids.
- **Abb. 6:**: Klonierung genomischer P3HR1 DNA ohne Verpackungssignale (TR) in E. coli mit Hilfe eines F-Faktor-Plasmids.
- **Abb. 7:**: Klonierung genomischer P3HR1 DNA in E. coli mit Hilfe eines F-Faktor-Plasmids.
- **Abb. 8:**: Einführen von Mutationen in F-Faktor klonierte genomische EBV DNA durch Allelaustausch
- **Abb. 9:**: Einführen von Mutationen in F-Faktor klonierte genomische EBV DNA durch selektive Integration

Es wurden in zwei verschiedene Zellinien, die latent mit zwei verschiedenen EBV Stämmen infiziert sind (B95.8 und P3HR1/HH514), ein Abschnitt des E. coli Plasmids F-Faktor eingeführt, der die Replikationsfunktionen und einen selektionierbaren Marker für E. coli umfaßt. Dieser lineare Abschnitt des F-Faktor Plasmids wurde jeweils links und rechts mit EBV Abschnitten flankiert, so daß der F-Faktorabschnitt über zwei homologe Rekombinationsereignisse in das endogene EBV Genom in den Zellinien targetiert werden kann. Zusätzlich zu den flankierenden EBV Abschnitten enthält das DNA Fragment mindestens ein Markergen, das zur Selektion (z. B. Hygromycinphosphotransferase) oder phänotypischen Charakterisierung (z. B. Green Fluorescence Protein, GFP) in eukaryonten Zellen geeignet ist. Dieses Konstrukt wird in EBV-infizierte Zellen transfiziert, es kommt zur homologen Rekombination mit dem endogenen EBV Genom und der zielgerichteten Integration des F-Faktor Abschnitts zusammen mit dem Markergen. Die so veränderte Zellinien tragen nun ein rekombinanten EBV Genom, das in einem Shuttlesystem in E. coli transferiert werden kann. Das rekombinante EBV Genom repliziert in E. coli über den F-Faktor Anteil, der auch den prokaryonten Selektionsmarker trägt. Weitere genetische Veränderungen des rekombinanten EBV Genoms können nun mit herkömmlichen genetischen Techniken in E. coli stattfinden.

Das rekombinante EBV Genom kann aus E. coli isoliert werden und die DNA Moleküle können über herkömmliche Techniken präpariert werden. Diese DNA Moleküle werden nun über DNA Transfertechniken in eukaryonte Zellen stabil oder transient eingeführt. Die rekombinanten EBV Genome replizieren extrachromosomal in diesen Zellen wie in den latent infizierten Zellinien 895.8 und P3HR1/HH514. Spontan oder nach chemischer oder anderweitiger Induktion der lytischen Phase von EBV kommt es zur Synthese von rekombinanten Epstein-Barr Virionen, die in den Zellkulturüberstand freigesetzt werden und die für weitere Anwendungen zur Verfügung stehen.

### Verfahren zur Herstellung

In das Genom des B95.8 Virus wurde mit dem oben beschriebenen Verfahren ein F-Faktor-Abschnitt eingeführt, der den eukaryonten Selektionsmarker Hygromycinphosphotransferase und das phänotypische Markergen GFP neben den funktionellen F-Faktor-Komponenten enthält. Das rekombinante B95.8 Genom wurde erzeugt, in E. coli transferiert und rekombinante EBV DNA aus E. coli präpariert. Diese DNA wurde in verschiedene EBV-negative Zellen (293, EBV-negative Akata, neuronale Zellen) eingeführt. Die Induktion des lytischen Zyklus führt zur Freisetzung von infektiösen Virionen, die als genetische Information rekombinantes EBV Genom enthalten. Diese rekombinanten EBV Genome sind z. B. in der Lage, primäre humane B-Lymphozyten zu immortalisieren.

In das Genom des P3HR1/HH514 Virus wurden in zwei unabhängigen Experimenten zwei verschiedene F-Faktor-Abschnitte in zwei unterschiedliche Genomorte eingeführt. Die Targetierung für diese Genomorte erfolgte durch unterschiedliche flankierende EBV-Abschnitte. Es wurde in einem Experiment ein weiteres EBV Gen eingeführt, in dem zweiten Experiment wurde ein EBV Abschnitt durch die Wahl der flankierenden EBV-Abschnitte gezielt deletiert.

Die rekombinanten Genome des B95.8 als auch des P3HR1/HH514 Virus wurde weiter in E. coli durch Deletionen oder Einfügen neuer Gene modifiziert.

### Ausführungsbeispiele

### 1. Klonierung eines voll funktionsfähigen Genoms des B95.8 Stammes

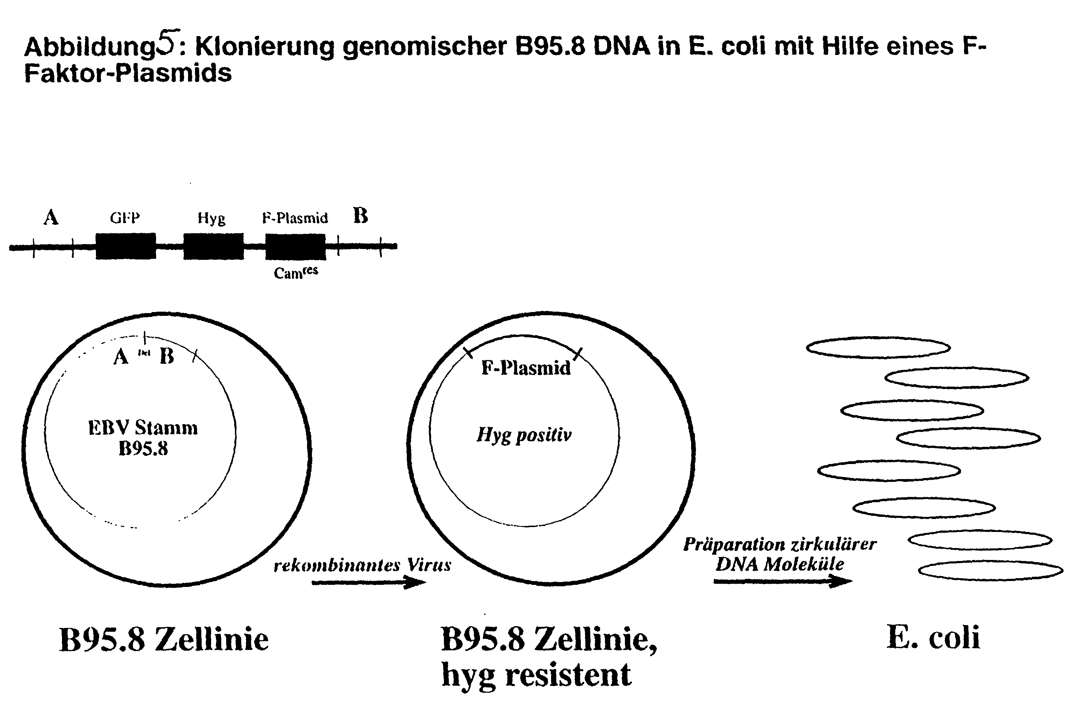

Ein F-Faktor Plasmid, mit p1944.12 bezeichnet (Tabelle 1), wurde in E. coli über konventionelle DNA-Klonierungstechniken hergestellt. Wie in Abbildung 5 schematisch gezeigt, enthält das Plasmid zwei Abschnitte A und B, die subgenomische Abschnitte des B95.8 Genoms darstellen Die Nukleotidsequenzkoordinaten dieser Abschnitte sind in Tabelle 1 angegeben. Die Abschnitte A und B flankieren das pMBO131 Replicon,(O'Connor *et al.,* 1989),. das den prokaryonten Replikationsursprung des F-Faktors zusammen mit dem selektionierbaren prokaryonten Markergen Chloramphenicolacetyltransferase (cam) umfaßt. Weiterhin ist das eukaryonte Markergen Hygromycinphosphotransferase (Hyg), das vom SV40 early Promotor/Enhancer exprimiert wird. und das Markergen Green Fluorescence Protein (GFP), das vom immediate early Promotor/Enhancer des humanen Cytomegalovirus ist, in dem Plasmid p1944.12 enthalten (Tabelle 1 und Abbildung 5). Funktionswichtige Teile des Plasmids p1944.12 sind die EBV-Abschnitte, der pMBO131 Anteil und das Gen Hyg, GFP sind optional.

Die Abschnitte A und B des B95.8 Genoms auf p1944.12 sind so gewählt, daß sie die natürliche Deletion im Genom von B95.8 links und rechts flankieren. Um dieses Plasmid in das Genom von B95.8 einzuführen, wurde es mit dem Restriktionsenzym Notl linearisiert und die freien DNA-Enden mit sogenannten Hairpin-Oligonukleotiden mit Hilfe von T4-DNA-Ligase versiegelt, um sie gegen Exonukleasen zu schützen. Die so veränderte Plasmid DNA wurde über Elektroporation in die B95.8 Zellen transfiziert und die Zellen in Gegenwart von 100 µg/ml Hygromycin selektioniert. Unter diesen Selektionsbedingungen überleben nur solche Zellen, die die DNA von p1944.12 aufgenommen und entweder in das Wirtszellgenom oder in die extrachromosomalen Genomkopien des B95.8 EBV Stammes integriert haben. Um zwischen diesen beiden Möglichkeiten zu unterscheiden, wurden Einzelzellklone mittels Gardella-Agarose-Gelen und anschließender Southernblothybridisierung untersucht. Es gelang, über diese Analysetechniken eine Reihe von Zellklonen zu identifizieren, die p1944.12 in die Genomkopien des B95.8 EBV Stammes integriert hatten.

Um diese genetisch veränderten EBV Genome in E.coli zu klonieren, wurde Plasmid DNA aus den Zellklonen isoliert, mit Hilfe von Elektroporationsmethoden in den E.coli Stamm DH10B transferiert und über die Resistenz gegen Chloramphenicol selectioniert. Die anschließende Isolierung von Plasmid DNA aus E.coli ergab DNA Präparationen, die nach Spaltung mit verschiedenen Restriktionsenzymen DNA Fragmente zeigten, die exakt der Komposition von B 98.8 DNA einschließlich integriertem p1944.12 Anteil entsprachen. Die Gesamtgröße dieser EBV Genome liegt über 170 kbp und wurde mit 2010 bezeichnet.

2010 DNA wurde im Mikrogrammmaßstab aus E.coli isoliert und über verschiedene DNA Transfektionsmethoden in EBV-negative eukaryonte Zellen eingebracht. Diese Zellen können beispielsweise EBV-negative Akatazellen. 293- und Fibroblastenzellen oder - zellinien sein. Diese Zellen wurden unter angepaßten Hygromycinkonzentrationen selektioniert, um sicherzustellen, daß 2010 DNA in die Zellen stabil eingebracht worden war. Die lytische Phase von EBV wurde über verschiedene Techniken induziert, z. B. durch Transfektion des BZLF1 Expressionsplasmids pCMV-BZLF1,(Hammerschmidt and Sugden. 1988). Die Induktion der lytischen Phase von EBV in diesen Zellen führt zur Freisetzung von infektiösen Viren, die zur Immortalisation von primären menschlichen B-Lymphozyten in der Lage sind. Um diese Eigenschaft von 2010 EBV nachzuweisen, wurde zellfreier Überstand aus lytisch induzierten Zellen gewonnen und zur Infektion von primären menschlichen B-Lymphozyten eingesetzt. Vier bis sechs Wochen nach Infektion dieser Zellen konnten permant proliferierende Zellinien etabliert werden, die 2010 EBV DNA enthielten, wie über verschiedene Techniken (Southern-Blot-Hybridisierung, PCR-Analyse) nachgewiesen wurde.

### 2. Klonierung eines verpackungsdefekten P3HR1/HH514 Genoms in E. coli

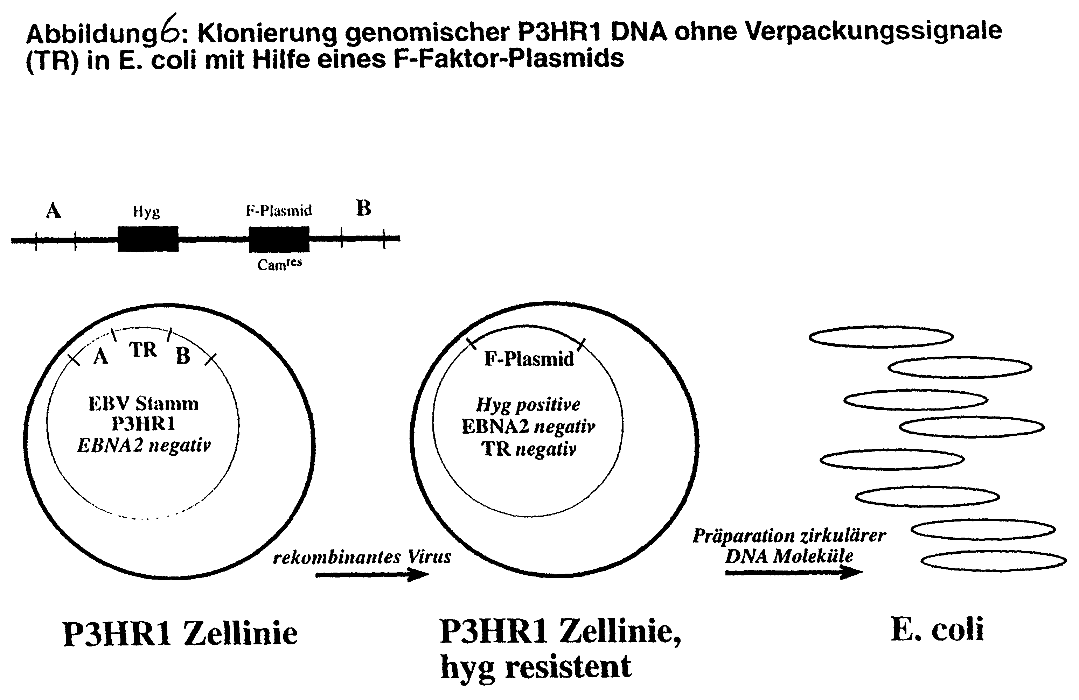

Ein F-Faktor Plasmid, mit p2061.2 bezeichnet (Tabelle 1), wurde in E. coli über konventionelle DNA-Klonierungstechniken hergestellt. Wie in Abbildung 6 schematisch gezeigt, enthält das Plasmid zwei Abschnitte A und B, die subgenomische Abschnitte des P3HR1/HH514 Genoms darstellen. Die Nukleotidsequenzkoordinaten dieser Abschnitte sind in Tabelle 1 angegeben. Die Abschnitte A und B flankieren das pMBO131 Replicon .(O'Connor *et al*., 1989)., das den prokaryonten Replikationsursprung des F-Faktors zusammen mit dem selektionierbaren prokaryonten Markergen Chloramphenicolacetyltransferase (cam) umfaßt. Weiterhin ist das eukaryonte Markergen Hygromycinphosphotransferase (Hyg), das vom SV40 early Promotor/Enhancer exprimiert wird, in dem Plasmid p2061.2 enthalten (Tabelle 1 und Abbildung 6). Funktionswichtige Teile des Plasmids p2061.2 sind die EBV-Abschnitte, der pMBO131/F-Faktor-Anteil und das Gen Hyg.

Die Abschnitte A und B des P3HR1/HH514 Genoms auf p2061.2 sind so gewählt, daß sie die 'terminal repeats' im Genom von P3HR1/HH514 links und rechts flankieren. Die 'terminal repeats' (TR) sind u.a. dadurch charakterisiert, daß sie die Signalsequenzen tragen, die für die Verpackung von viraler genomischer DNA in EBV Kapside notwendig sind. Die Konstruktion von p2061.2 hat zum Ziel, die TR Signalsequenzen zu deletieren und durch die Anteile pMBO131 und Hyg von p2061.2 zu ersetzen. Um p2061.2 in das Genom von P3HR1/HH514 einzuführen, wurde es mit dem Restriktionsenzym Sacl linearisiert und die freien DNA-Enden mit sogenannten Hairpin-Oligonukleotiden mit Hilfe von T4-DNA-Ligase versiegelt, um sie gegen Exonukleasen zu schützen. Die so veränderte Plasmid DNA wurde über Elektroporation in die P3HR1/HH514 Zellen transfiziert und die Zellen in Gegenwart von 200 µg/ml Hygromycin selektioniert. Unter diesen Selektionsbedingungen überleben nur solche Zellen, die die DNA von p2061.2 aufgenommen und entweder in das Wirtszellgenom oder in die extrachromosomalen Genomkopien des P3HR1/HH514 EBV Stammes integriert haben. Um zwischen diesen beiden Möglichkeiten zu unterscheiden, wurden Einzelzellklone mittels Gardella-Agarose- Gele und anschließender Southernblothybridisierung untersucht. Es gelang, über diese Analysetechniken eine Reihe von Zellklonen zu identifizieren, die p2061.2 in die Genomkopien des P3HR1/HH514 EBV Stammes integriert hatten.

Um diese genetisch veränderten EBV Genome in E. coli zu klonieren, wurde Plasmid DNA aus den Zellklonen isoliert, mit Hilfe von Elektroporationsmethoden in den E. coli Stamm DH10B transferiert und über die Resistenz gegen Chloramphenicol selektioniert. Die anschließende Isolierung von Plasmid DNA aus E. coli ergab DNA Präparationen, die nach Spaltung mit verschiedenen Restriktionsenzymen DNA Fragmente zeigten, die exakt der Komposition von P3HR1/HH514 genomischer DNA ohne den TR Signalsequenzen, aber einschließlich integriertem p2061.2 Anteil, entsprachen. Die Gesamtgröße dieser EBV Genome liegt über 170 kbp und wurde mit 2087.2a bezeichnet.

2087.2a DNA wurde im Mikrogrammaßstab isoliert und über verschiedene DNA Transfektionsmethoden in EBV-negative eukaryonte Zellen eingebracht. Diese Zellen können beispielsweise EBV-negative Akatazellen, 293- und Fibroblastenzellen oder - zellinien sein. Diese Zellen wurden unter angepaßten Hygromycinkonzentrationen selektioniert, um sicherzustellen, daß 2087.2a DNA in die Zellen stabil eingebracht worden war. Die lytische Phase von EBV wurde über verschiedene Techniken induziert, z. B. durch Transfektion des BZLF1 Expressionsplasmids pCMV-BZLF1,(Hammerschmidt and Sugden, 1988). Im Gegensatz zu dem unter 1, beschriebenen Ausführungsbeispiel führt die Induktion der lytischen Phase von EBV in diesen Zellen nicht zur Freisetzung von infektiösen Viren, da die Verpackung von EBV genomischer DNA durch das Fehlen der TR-Verpackungssignale unterbleibt. Diese Zellinien stellen aber alle viralen Funktionen zur Verpackung von DNA Molekülen in trans zur Verfügung, die z. B. für die Enkapsidierung von p554 (Hammerschmidt and Sugden, 1989), oder mini-EBV-Plasmiden,(Kempkes *et al*., 1995a; Kempkes *et al*., 1995b), notwendig sind, ohne daß sogenanntes Helfervirus freigesetzt wird (Hammerschmidt and Sugden, 1989).

### 3. Klonierung eines P3HR1/HH514 Genoms mit genetischer Komplementation in E. coli.

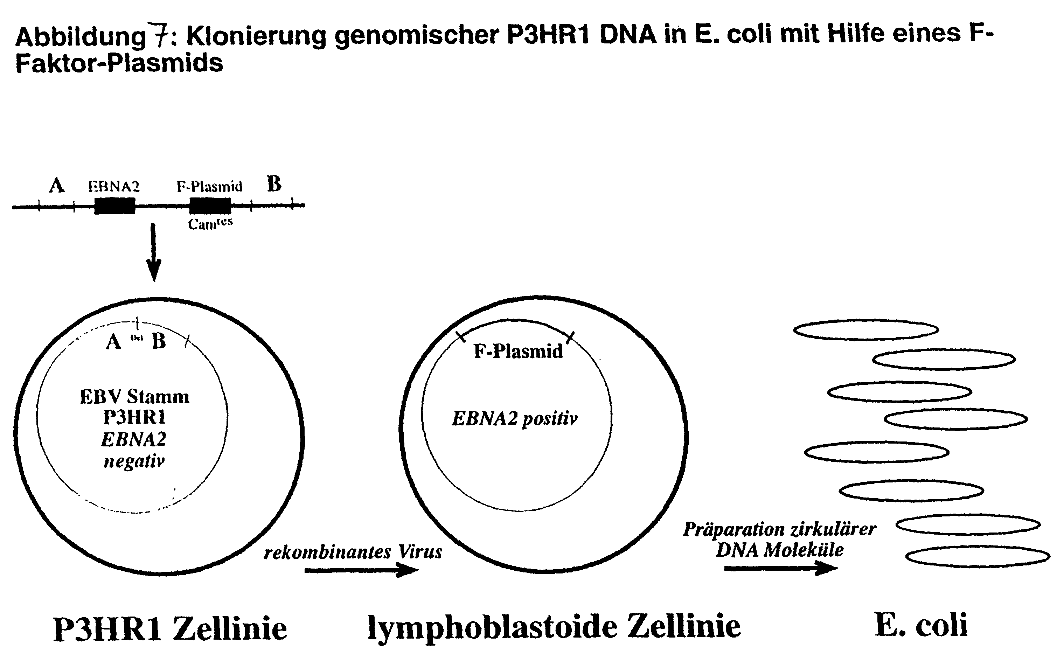

Ein F-Faktor Plasmid, mit p1820.15 bezeichnet (Tabelle 1) wurde in E. coli über konventionelle DNA-Klonierungstechniken hergestellt. Wie in Abbildung 7 schematisch gezeigt, enthält das Plasmid zwei Abschnitte A und B, die subgenomische Abschnitte des B95.8 Genoms darstellen. Die Nukleotidsequenzkoordinaten dieser Abschnitte sind in Tabelle 1 angegeben. Die Abschnitte A und B flankieren das pMBO131 Replicon,(O'Connor *et al*., 1989)., das den prokaryonten Replikationsursprung des F-Faktors zusammen mit dem selektionierbaren prokaryonten Markergen Chloramphenicolacetyltransferase (cam) umfaßt. Weiterhin sind als funktionelle Abschnitte in dem Plasmid p1820.15 das EBV Gen EBNA2 und zwei Exons des EBV Gens EBNA-LP enthalten (Hammerschmidt und Sugden, 1989) (Tabelle 1 und Abbildung 7). Funktionswichtige Teile des Plasmids p1820.15 sind die flankierenden EBV-Abschnitte, das Gen EBNA2 und Anteile des Gens EBNA-LP und der pMBO131 Anteil.

Die Abschnitte A und B des 895.8 Genoms auf p1820.15 sind so gewählt, daß sie die Deletion im Genom von P3HR1/HH514 links und rechts flankieren, die zwei Exons von EBNA-LP und das gesamte EBNA2 Gen umfaßt. Um dieses Plasmid in das Genom von P3HR1/HH514 einzuführen, wurde es mit dem Restriktionsenzym Nhel linearisiert und die freien DNA-Enden mit sogenannten Hairpin-Oligonukleotiden mit Hilfe von T4-DNA-Ligase versiegelt, um sie gegen Exonukleasen zu schützen. Die so veränderte Plasmid DNA wurde über Elektroporation in die P3HR1/HH514 Zellen transfiziert zusammen mit dem Expressionsplasmid pCMV-BZLF1, das den lytischen Zyklus von EBV in diesen Zellen induziert. Zellfreier Kulturüberstand wurde gewonnen und zur Infektion von primären menschlichen B-Lymphozyten eingesetzt. Vier bis sechs Wochen nach Infektion dieser Zellen konnten permanent proliferiende Zellinien etabliert werden. Die Deletion in P3HR1/HH514, die das gesamte EBNA2 Gen und zwei Exons des EBNA-LP Gens umfaßt, unterbindet die B-Zell-Immortalisation. Die Abschnitte auf dem Plasmid p1820.15 komplementieren die Deletion im Genom von P3HR1/HH514 Virus über die homologen Rekombination zwischen P3HR1/HH514 DNA und p1820.15 DNA, ähnlich wie beschrieben .(Hammerschmidt and Sugden. 1989).. Die homolog rekombinierten EBV Genome zeichnen sich durch die wiedererlangte Fähigkeit aus. primäre menschliche B-Lymphozyten zu immortalisieren. Gleichzeitig kommt es zur Integration des F-Faktoranteils in p1820.15, so daß das Replicon pMBO131 Bestandteil des rekombinanten EBV Genoms wird.

Um diese genetisch veränderten EBV Genome in E. coli zu klonieren, wurde Plasmid DNA aus den Zellklonen isoliert, mit Hilfe von Elektroporationsmethoden in den E. coli Stamm DH10B transferiert und über die Resistenz gegen Chloramphenicol selektioniert. Die anschließende Isolierung von Plasmid DNA aus E. coli ergab DNA Präparationen, die nach Spaltung mit verschiedenen Restriktionsenzymen DNA Fragmente zeigten, die exakt der Komposition von P3HR1/HH514 genomischer DNA einschließlich dem integriertem Anteil von p1820.15 entsprachen. Die Gesamtgröße dieser EBV Genome liegt über 170 kbp und wurde mit 1947 (K-Klon) bezeichnet.

### 4. Einführen von Mutationen in F-Faktor klonierte genomische EBV DNA durch Allelaustausch

Die Klonierung von genomischer EBV DNA in E. coli erlaubt die einfache genetische Modifikation durch etablierte bzw. an die erfindungsgemäßen, speziellen Bedingungen angepaßte Methoden.

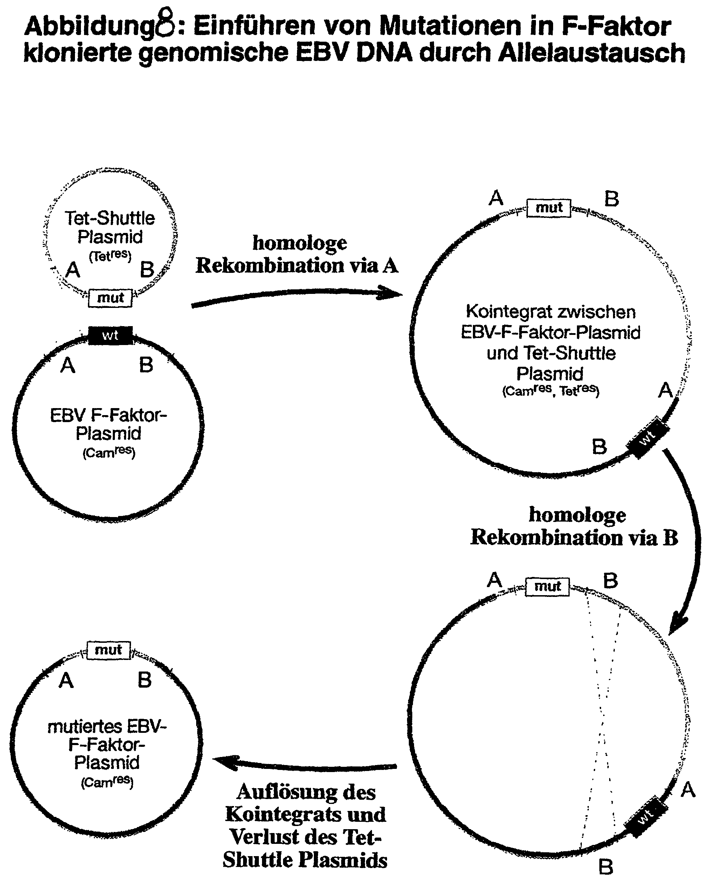

Zum Austausch der Verpackungssignale in dem klonierten EBV Genom 1947 (Tabelle 1) wurde das rekombinante Plasmid p2060.1 in E. coli hergestellt, das aus folgenden Komponenten besteht (siehe Abb. 8):
1. Einem prokaryonten Vektorplasmid mit temperatursensitivem Replikationsursprung (Tet-Shuttle Plasmid) und dem prokaryonten Selektionsmarker Tetracyclinresistenz, z. B. auf der Basis von pMBO96.(O'Connor *et al*., 1989).
2. Flankierenden Abschnitten A und B, die die EBV Sequenzen des P3HR1/HH514 Stammes von Nukleotidposition #165,840 bis #169,924 (A) und #1 bis #3955 (B) enthalten.
3. Dem eukaryonten Selektionsmarker Hygromycinphosphotransferase, exprimiert vom SV40 early Promoter/Enhancer. Dieses Gen liegt zwischen den EBV Abschnitten A und B und ist in Abb. 8 mit "mut" bezeichnet.

Das F-Faktor-Plasmid 1947 (Tabelle 1) wird in einen rekombinationskompetenten (recA+) E. coli Stamm transfektiert, und die Transfektanten werden durch ihre Chloramphenicolresistenz identifiziert. In einem zweiten Schritt wird das Tet-Shuttle-Plasmid p2060.1 in die 1947 tragenden Transfektanten transfektiert und die Bakterien bei 30°C auf die Doppelresistenz gegen Chloramphenicol und Tetracyclin selektiert. Die beiden Plasmide replizieren unabhängig voneinander in derselben E. coli Zelle. Die homologe Rekombination via A (kann auch via B erfolgen) wird durch Erhöhung der Temperatur auf 42°C und Selektion auf Resistenz gegen Chloramphenicol und Tetracyclin erzwungen, da das Tet-Shuttle-Plasmid p2060.1 bei dieser Temperatur nicht mehr replizieren kann. Ein Kointegrat entsteht wie in Abb. 8 gezeigt. Mit einer weiteren homologen Rekombination, diesmal via B. erfolgt die Auflösung des Kointegrats und der Verlust des Tet-Shuttle-Plasmids, so daß die Veränderung "mut" (in diesem Fall das Gen Hygromycinphosphotransferase) die EBV Sequenz "wt" (in diesem Fall die Verpackungssignale TR) ersetzt. Der letzte Schritt erfolgt nach statistischer Verteilung, d. h. bei der Auflösung des Kointegrats kann entweder die Mutation "mut" eingeführt werden (wie in Abb. 8 gezeigt) oder die Ausgangssitutation "wt" wieder entstehen.

Vorteil dieser Methode gegenüber der unter 5, beschriebenen ist die Möglichkeit, ohne Einführen von weiteren Fremdsequenzen ganz gezielt auch einzelne Nukleotide im klonierten EBV-Genom auszutauschen.

### 5. Einführen von Mutationen in F-Faktor klonierte genomische EBV DNA durch selektive Integration

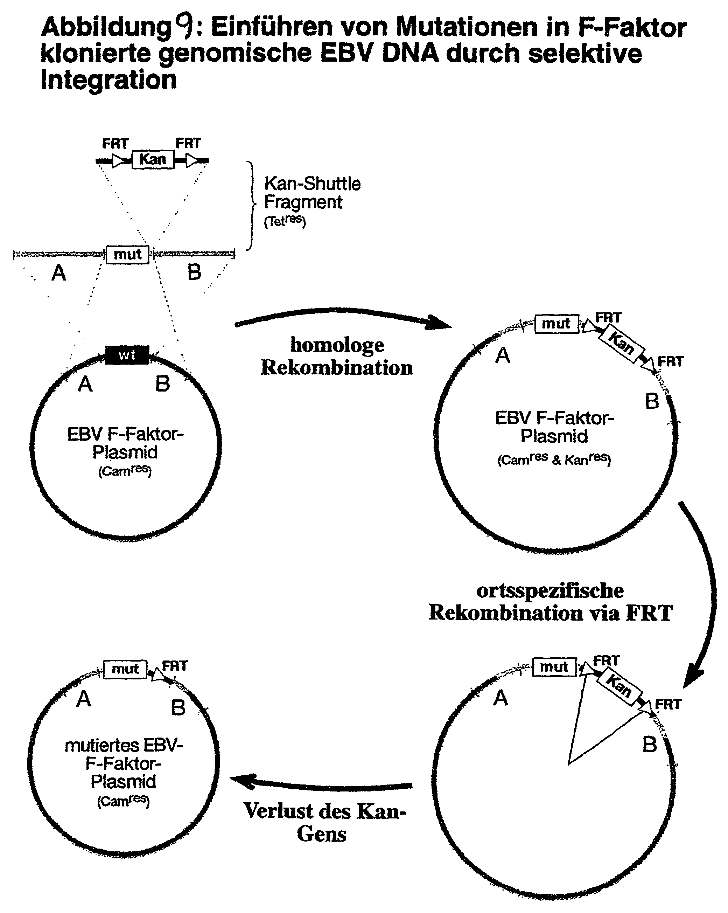

Eine zweite, alternative Methode zur genetischen Modifikation von F-Faktor klonierter genomischer EBV DNA besteht in der Integration von linearen DNA Fragmenten durch homologe Rekombination, wie in Abbildung 9 schematisch gezeigt.

Zur Mutation des EBV Gens LMP2A wird zuerst in einem gewöhnlichen, von pBR abgeleiteten Plasmid ein Kan-Shuttle-Fragment kloniert, wie in Abb. 9 gezeigt. Das Kan-Shuttle-Fragment in dem Plasmid p2120 enthält zwei EBV Abschnitte A und B, die die EBV Sequenzen des B95.8 Stammes von Nukleotidposition #163,473 bis #166,180 (A) und #166,938 bis #172,281/#1 bis #649 (B) enthalten. Die in Abb. 9 bezeichnete Mutation "mut" besteht aus zwei sogenannten loxP Sequenzmotiven, die ein Exon des EBV Gens LMP2A (EBV Nukleotidpositionen #166,181 bis #166,937) flankieren. Anschließend an eine der beiden loxP Sequenzmotive ist ein mit FRT Sequenzmotiven begrenzter Selektionsmarker für die Resistenz gegen das Antibiotikum Kanamycin kloniert. Das Fragment, wie in Abb. 5 oben links dargestellt, wird durch Restriktionsenzyme vom pBR Anteil getrennt und die lineare Kan-Shuttle-Fragment-DNA isoliert. Die Kotransfektion dieses linearen DNA-Fragments zusammen mit dem F-Faktor klonierten EBV Genom 2010 erfolgt in einen E. coli Stamm, der vorzugsweise Exonuklease V negativ ist; z. B. den E. coli Stamm BJ5183 .(Hanahan, 1983). Die Selektion der transfektierten Bakterien auf Resistenz gegen Chloramphenicol und Kanamycin erzwingt die homologe Rekombination zwischen beiden DNA Molekülen und ergibt idealerweise das oben rechts gezeigte EBV F-Faktor-Plasmid. Das Kanamycinresistenzgen kann durch ortspezifische Rekombination via FRT über die Rekombinase FLP in E. coli entfernt werden (Cherepanov and Wackernagel, 1995). Als Ergebnis entsteht ein mutiertes EBV-F-Faktor-Plasmid. Der prinzipielle Unterschied zu der Situation unter 4, ist der Verbleib von Fremdsequenzen, nämlicher eines FRT Sequenzmotivs.

Antibiotikaresistenzmarker und Sequenzen, die für die ortspezifische Rekombination eingesetzt werden, sind beliebig wählbar, was gleichermaßen auch für den Ansatz unter 4, gilt.

Des weiteren ist die Erstellung einer Genombank denkbar, die durch Transposon-vermittelte Mutagenese in E. coli hergestellt werden kann und die damit eine beliebig große Anzahl an individuell mutierten EBV Genomen repräsentiert. Durch die zufällige Insertion der Transposons in die klonierten EBV Genome kommt es an der Insertionsstelle zur Mutagenese, z. B. zur Unterbrechung des Leserahmens eines EBV Gens. Der Vorteil einer solchen EBV-Genombank wäre die Möglichkeit, auf bestimmte Phänotypen biologisch selektionieren zu können. Eine Genombank mit mutierten herpesviralen Genomen könnte z. B. als Screeningsystem für antivirale Substanzen eingesetzt werden.

Das Verfahren kann somit noch wie folgt abgewandelt werden:
(f) Einführen des rekombinanten Herpes-Virus-Vektor-Genoms in eine Prokaryontenzelle;
(g) Einführen eines Plasmids in die Prokaryontenzelle, das zumindest, in funktioneller Anordnung, eine DNA-Sequenz enthält, die im Vergleich zu der auf dem Herpes-Virus-Vektor-Genom vorliegenden homologen Sequenz eine Mutation enthält, und ein selektierbares Markergen, flankiert von DNA-Abschnitten in einer solchen Länge, die eine Rekombination mit dem Herpes-Virus-Vektor-Genom ermöglichen;
(h) Integration des unter (g) beschriebenen Abschnitts durch Rekombination in das Herpes-Virus-Genom:
(i) wahlweise Aufreinigen und Isolieren des rekombinanten Herpes-Vektors.

In einer weiteren Ausführungsform enthält das Plasmid im Schritt (g) weiterhin von einer Rekombinase erkennbare Sequenzmotive, und vor dem Schritt (e) wird das durch die Rekombination eingeführte Resistenzgen durch ortsspezifische Rekombination über die von der Rekombinase erkannten Sequenzmotive wieder entfernt.

Entfernung des prokaryonten Replikons und anderer Fremdanteile aus herpesviralen Genomen kloniert in E.coli.

Bei der Anwendung von Genvektoren ist es wünschenswert, den Anteil der genetischen Information, der in die Zielzelle eingebracht wird, so klein wie möglich zu halten und auf das wesentliche zu beschränken. Insbesondere sind subgenomische Abschnitte unerwünscht, die prokaryonte Markergene wie Antibiotikaresistenzen tragen oder aber ein potentielles Sicherheitsrisiko darstellen, da sie Anlaß für homolge Rekombinationen mit verschiedenen Bakterien sein könnten. Die mit Hilfe eines F-Faktor Replikons klonierten EBV-Genome stellen ein solch hybrides Molekül dar. Der F-Faktoranteil ist bei der Propagierung des DNA-Moleküls in E. coli essentiell, er ist aber völlig funktinslos in der eukaryonten Zelle, in die das in E. coli modifizierte EBV/F-Faktor Replikon eingeführt wird, um genetisch modifiziertes EBV herzustellen.

Es wurde eine überraschend einfache Methode gefunden, um diesen F-Faktoranteil gänzlich und ohne weitere genetische Manipulation zu entfernen.

Bei der Klonierung eines voll funktionsfähigen Genoms des B95.8 Stammes in E. coli wurde ein F-Faktor Plasmid verwendet, das mit p1944.12 bezeichnet wurde (Tabelle 1). Es wurde in E. coli über konventionelle DNA-Klonierungstechniken hergestellt. Wie in Abbildung 5 schematisch gezeigt, besteht das Plasmid aus zwei Abschnitten A und B, die subgenomische Abschnitte des B95.8 -Genoms darstellen. Die Nukleotidsequenzkoordinaten dieser Abschnitte sind in Tabelle 1 angegeben; sie erstrecken sich von EBV-Koordinate #143.458 bis #152.636 im Abschnitt A und von #149.930 bis #159.880 im Abschnitt B. Die Abschnitte A und B flankieren das pMBO131-Replicon (O'Connor et al., 1989), das den prokaryonten Replikationsursprung des F-Faktors zusammen mit dem selektionierbaren, prokaryonten Markergen Chloramphenicolacetyltransferase (cam) und anderen Genen umfaßt. Die beiden EBV-Anteile sind so gewählt, daß sie teilweise die gleichen DNA-Sequenzabschnitte tragen, die im konkreten Beispiel eine partielle Duplikation von 2,7 kbp darstellen (von #149.930 bis #152.636). Das aus diesen Experimenten in E. coli klonierte F-Faktor-Plasmid wurde mit 2010 bezeichnet (Tabelle 1). Nach Transfektion der 2010 DNA in 293 Zellen kommt es spontan zu homolgen Rekombinationsereignissen zwischen den duplizierten Anteilen in den Abschnitten A und B in diesen Zellen und zur Deletion aller Sequenzen, die aus den prokaryonten Klonierungsschritten stammen.

Als weitere denkbare Anwendungen hier einige Beispiele:
- Etablierung von gezielt veränderten Herpes-Viren, denen bestimmte Virulenzgene fehlen und die als gezielt attenuierte Vakzinestämme eingesetzt werden können. Als Virulenzgene kommen im Fall vor EBV z.B. EBNA2, LMP1, LMP2A, u. a. in Frage, die für die Attenuierung eines solchen Vakzinestammes deletiert werden könnten.
- Generierung von rekombinanten EBV Vektoren, die in Verpackungszellinien mit einer EBV-Hülle versehen werden und die zum Gentransfer in Zellen des Menschen oder anderer Mammalier in vitro oder in vivo eingesetzt werden können. Diese Verpackungszellinien enthalten stabil ein EBV Genom oder dessen wichtige Teile, die für die Produktion viraler Strukturproteine nötig sind, um EBV Vektoren zu verpacken. Die Verpackungszellinien werden vorzugsweise mit einem EBV Genom versehen, dem die eigenen Verpackungssignale fehlen (siehe Abb. 6 und Ausführungen), um das Freisetzen von unerwünschten, sogenannten Helferviren zu verhindern. Die verpackbaren EBV Vektoren können auf herkömmlichen rekombinanten DNA Plasmiden beruhen, die alle Elemente enthalten, die für die Amplifikation ihrer DNA (oriLyt), deren Verpackung (TR), und der stabilen extrachromosomalen Existenz in der Rezipientenzelle (oriP und EBNA1) nötig sind. Zusätzlich können diese Vektoren therapeutisch interessante Gene, z.B. Faktor VIII, im Sinne einer Genkorrektur tragen.

Darüber hinaus kann die Affinität dieser Vektoren für bestimmte Zielzellen verändert werden. EBV Glykoproteine, die für die Infektion bestimmter Zellen (natürlicherweise B-Zellen und einige Epithelzellen) verantwortlich sind, können durch Mutation der F-Faktor klonierten EBV Genome in E. coli verändert oder gegen andere ausgetauscht werden. Ein Beispiel für einen erweiterten Zelltropismus wäre das Vesikulostomatitisvirus-Glykoprotein G, ein Beispiel für den extrem spezifischen Zelltropismus für fast ausschließlich pluripotente hämatopoetische Stammzellen wäre der Ligand für den Stem Cell Factor Receptor auf der Stammzelle, der membranständige Stem Cell Factor Ligand (mSCF-ligand). Dieses sogenannte Pseudotyping ist besonders für herpesvirale Vektoren attraktiv, da die für die Infektiosität wichtigen Glykoproteine in einer von der Kernmembran abgeleiteten Phospholipidmembran eingebettet sind. Diese Membran ist nicht wesentlich strukturiert und umhüllt das streng geometrische Kapsid des Virions nur locker. Im Gegensatz zu vielen kleineren Viren (z.B. Adenovirus, Adenoassoziiertes Virus (AAV), Retroviren) ist die Hülle von Herpesviren und anderen großen DNA Viren flexibel und verlangt keine bestimmten Proteinkonfigurationen oder -faltungen, die anderenfalls die Virusreifung und -assemblierung verhindern.

### Vorteile gegenüber dem Stand der Technik

Das beschriebene Verfahren erlaubt beispielsweise die Herstellung von rekombinanten Herpesvirusgenomen, die z.B. in E. coli klonal vermehrt werden können. Die Klonierung dieser Virusgenome z.B. in E. coli erlaubt die einfache Modifikation durch gezielte Veränderungen viraler Gene, ihre Deletion oder Hinzufügen von weiteren Genen, die von Interesse sind. Die so hergestellten viralen Genome weisen je nach genetischer Modifikation neue Eigenschaften auf, die z. B. die Expression von Proteinen in den jeweiligen Zielzellen der Viren erlauben.

Die in E. coli klonierten viralen Genome stellen ein Produkt dar, das z. B. für die Enkapsidierung von rekombinanten Plasmiden oder subgenomischen viralen Abschnitten geeignet ist.

### LITERATURLISTE

Baer, R., Bankier, A. T., Biggin, M. D., Deininger, P. L., Farrell, P. J., Gibson, T. J., Hatfull, G., Hudson, G. S., Satchwell, S. C., Seguin, C., Tufnell, P S., und Barell, B. G. (1984). DNA sequence and expression of the B95-8 Epstein-Barr virus genome Nature (London) *310*, 207-211.
Cohen, J. I., Wang, F., Mannick, J., und Kieff, E. (1989). Epstein-Barr virus nuclear protein 2 is a key determinant of lymphocyte transformation. Proc. Natl. Acad. Sci. U S A *86*, 9558-9562.
Hammerschmidt, W., und Sugden, B. (1988). Identification and characterization of *oriLyt*, a lytic origin of DNA replication of Epstein-Barr virus. Cell *55,* 427-433.
Hammerschmidt, W., und Sugden, B. (1989). Genetic analysis of immortalizing functions of Epstein-Barr virus in human B lymphocytes. Nature (London) *340,* 393-397
Hammerschmidt, W., und Sugden, B. (1995a). Immortalized lymphocytes for production of viral-free proteins (USA, Canada, Mexico, Japan.
Kempkes, B., Pich, D., Zeidler, R., und Hammerschmidt, W. (1995a). Immortalization of human primary B-lymphocytes *in vitro* with DNA. Proc. Natl. Acad. Sci. USA *92*, 5875-5879.
Kempkes, B., Pich, D., Zeidler, R., Sugden, B., und Hammerschmidt, W. (1995b). Immortalization of human B-lymphocytes by a plasmid containing 71 kpb of Epstein-Barr viral DNA. J. Virol. *69*, 231-238.
Moore, M. D., Cooper, N. R., Tack, B. F., und Nemerow, G. R. (1987). Molecular cloning of the cDNA encoding the Epstein-Barr virus/C3d receptor (complement receptor type 2) of human B lymphocytes. Proc. Natl. Acad. Sci. USA *84*, 9194-9198.
Nemerow, G. R., Houghten, R. A., Moore, M. D., und Cooper, N. R. (1989). Identification of an epitope in the major envelope protein of Epstein- Barr virus that mediates viral binding to the B lymphocyte EBV receptor (CR2). Cell *56*, 369-377.
Yates, J. L., Warren, N., und Sugden, B. (1985). Stable replication of plasmids derived from Epstein-Barr virus in various mammalian cells. Nature (London) *313*, 812-815.
Zimmermann. J., und Hammerschmidt, W. (1995). Structure and role of the terminal repeats of Epstein-Barr virus in processing and packaging of virion DNA. J. Virol. *69*, 3147-3155.
Cherepanov, P.P. und Wackernagel, W. (1995). Gene disruption in Escherichia coli: TcR and KmR cassettes with the option of Flp-catalyzed excision of the antibioticresistance determinant. Gene, *158*, 9-14.
Hammerschmidt, W und Sugden, W.M. (1993) US-A-5 194 601
Hammerschmidt, W. und Sugden, B. (1995) EP-A-0 694 613
Hanahan, D. (1983). Studies on transformation of Escherichia coli with plasmids. J. Mol. Biol., 166, 557-580.
O'Connor, M., Peifer, M. und Bender, W. (1989). Construction of large DNA segments in Escherichia coli. Science, 244, 1307-1312.
Firth, N., Ippen-Ihler, K., und Skurray, R.A. (1996). Structure and function of the F factor and mechanism of conjugation. In Escherichia coli and Salmonella, F.C. Neidhardt, R. Curtiss III, J.L. Ingraham, E.C.C. lin, K.B. Low, B. Magasnik, W.S. Reznikoff, M. Riley, M. Schaechter und H.E. Umbarger, eds (Washington, D.C.: American Society for Microbiology Press, 2377-2401).
Shizuya, H., Birren, B., U.J., Mancino, V., Slepak, T., Tachiiri, Y., und Simon, M. (1992). Cloning and stable maintenance of 300-kilobase-pair fragments of human DNA in Escherichia coli using an F-factor-based vector. Proc. Natl., Acad. Sci. USA 89, 8794-8797.

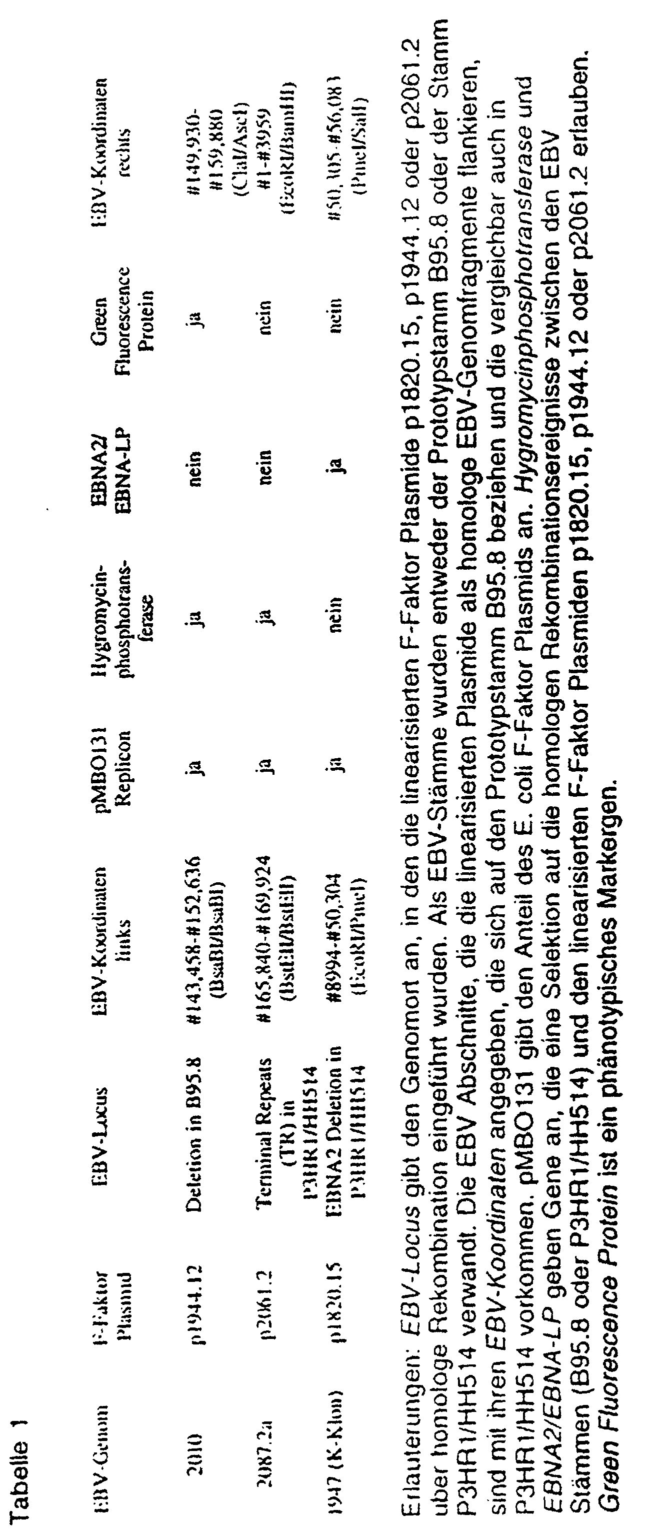

## Patentansprüche

1. Verfahren zur helfervirusfreien Verpackung einer Genvektor-DNA in die Viruspartikel eines Herpes-Virus mit den nachfolgenden Schritten:
(a) Einführen einer Herpes-Helfervirus-Vektor-DNA, auf einem oder zwei Molekülen, mit zumindest den nachfolgenden Merkmalen:
(α) einer Gesamtgröße ≥ 100 kbp;
(β) mit zumindest einer Mutation, die so ausgelegt ist, daß eine oder mehrere der cis-aktiven Signalsequenzen zur Verpackung der Helfervirus-Vektor-DNA nicht funktionell sind;
(γ) mit der Information zur Herstellung der Virus-Partikel des Herpes-Helfervirus, die kein Helfervirusgenom enthalten,
(δ) mit der Information für ein in eukaryonten Zellen selektierbares Markergen;
in eine Eukaryontenzelle;
(b) Etablieren einer Zelllinie, die die Herpes-Helfervirus-Vektor-DNA in stabiler Form enthält;
(c) Einführen einer zu verpackenden Genvektor-DNA mit zumindest
(α) einer cis-aktiven Signalsequenz zur Verpackung der Genvektor-DNA in ein Viruspartikel des Herpes- Helfervirus;
(β) einem Gen von Interesse
in die Eukaryontenzelle;
(d) Induktion der lytischen Phase des Herpes-Helfervirus und Produktion der für die Verpackung wichtigen Proteine des Herpes-Helfervirus;
(e) Verpackung der Genvektor-DNA in die Viruspartikel des Herpes-Helfervirus; wahlweise
(f) Freisetzen von die Genvektor-DNA enthaltenden Viruspartikeln; und/oder
(g) wahlweise Aufreinigen der Viruspartikel.

2. Verfahren nach Anspruch 1,
**dadurch gekennzeichnet, daß**
das Herpes-Helfervirus ausgewählt wird aus einer Gruppe, umfassend eine Genom-Größe von ≥ 120 kbp, ≥ 150 kbp oder ≥ 170 kbp.

3. Verfahren nach Anspruch 1 oder 2,
**dadurch gekennzeichnet, daß**
das Herpes-Virus ein Alpha-Herpes-Virus, ein Beta-Herpes-Virus oder ein Gamma-Herpes-Virus ist.

4. Verfahren nach Anspruch 3,
**dadurch gekennzeichnet, daß**
das Alpha-Herpes-Virus ein Herpes-Simplex-Virus (HSV) oder ein Varicella-Zoster-Virus (VZV) ist, das Beta-Herpes-Virus ein Cytomegalie-Virus (CMV) ist und das Gamma-Herpes-Virus ein Epstein-Barr-Virus (EBV) ist.

5. Verfahren nach einem oder mehreren der vorhergehenden Ansprüche,
**dadurch gekennzeichnet, daß**
die Herpes-Helfervirus-Vektor-DNA die Information für die Replikation des viralen DNA-Genoms ein prokaryontischen Zellen und wahlweise ein in prokaryontischen Zellen selektierbares Markergen umfaßt.

6. Verfahren nach einem oder mehreren der vorhergehenden Ansprüche,
**dadurch gekennzeichnet, daß**
die Herpes Helfervirus-Vektor-DNA EBV-DNA als Helfervirus-DNA enthält.

7. Verfahren nach einem oder mehreren der vorhergehenden Ansprüche,
**dadurch gekennzeichnet, daß**
als cis-aktive Signalsequenz die terminalen Repeatsequenzen des EBV, der lytische Replikationsursprung des EBV, der plasmidale Replikationsursprung oriP des EBV und/oder das EBNA1-Gen des EBV eingesetzt werden.

8. Verfahren nach einem oder mehreren der vorhergehenden Ansprüche,
**dadurch gekennzeichnet, daß**
das Markergen ein Antibiotikumresistenzgen oder ein Gen ist, das für ein durch Fluoreszenz nachweisbares Protein kodiert.

9. Verfahren nach einem oder mehreren der vorhergehenden Ansprüche,
**dadurch gekennzeichnet, daß**
als Eukaryontenzelle eine Humanzelle eingesetzt wird.

10. Verfahren nach einem oder mehreren der vorhergehenden Ansprüche,
**dadurch gekennzeichnet, daß**
als Gen von Interesse ein therapeutisch wertvolles Gen eingesetzt wird.

11. Verfahren nach einem oder mehreren der vorhergehenden Ansprüche,
**dadurch gekennzeichnet, daß**
in die Eukaryontenzelle ein Gen zur Beeinflußung des Zelltropismus des Herpes-Helfervirus eingeführt wird.

12. Verfahren nach Anspruch 11,
**dadurch gekennzeichnet, daß**
das den Zelltropismus beeinflussende Gen auf der Genvektor-DNA vorliegt.

13. Verfahren nach einem oder mehreren der vorhergehenden Ansprüche,
**dadurch gekennzeichnet, daß**
die Mutation Punktmutationen, mehrere Nukleotide betreffende Mutationen, Deletionen, Nukleotidaustausche und Additionen umfaßt.

14. Eukaryonten-Helferzelle zur helfervirusfreien Verpackung von Genvektor-DNA in die Viruspartikel eines Herpes-Helfervirus, zumindest enthaltend:
(a) eine Herpes-Helfervirus-Vektor-DNA ≥ 100 kbp, auf einem oder zwei Molekülen, mit einer Mutation, die so ausgelegt ist, daß eine oder mehrere der cis-aktiven Signalsequenzen zur Verpackung nicht-funktionell sind, mit der Information zur Herstellung der Viruspartikel, die kein Helfervirusgenom enthalten, und mit der Information für ein in eukaryonten Zellen selektierbares Markergen
(b) eine Genvektor-DNA mit zumindest
(α) einer cis-aktiven Signalsequenz zur Verpackung der Genvektor-DNA in ein Viruspartikel des Helfervirus;
(β) einem Gen von Interesse.

15. Eukaryonten-Helferzelle nach Anspruch 14,
**dadurch gekennzeichnet, daß**
die Herpes-Helfervirus-Vektor-DNA EBV-DNA als Helfervirus DNA enthält.

16. Eukaryonten-Helferzelle nach Anspruch 14 oder 15,
**dadurch gekennzeichnet, daß**
sie eine Humanzelle ist.

17. Viruspartikel oder Virus-DNA, erhältlich durch ein Verfahren nach einem oder mehreren der vorhergehenden Ansprüche.

## Claims

1. Method for helper virus-free packaging of a gene vector DNA into the viral particles of a Herpes virus comprising the following steps of:
a) Introduction of a Herpes helper virus vector DNA located on one or two molecules having at least the following features:
(α) a total size of ≥ 100 kbp;
(β) having at least one mutation designed to render one or more of the cis-acting signal sequences for packaging of the helper virus vector DNA non-functional;
(Y) having the information for the production of virus particles of the Herpes helper virus which do not contain a helper virus genome;
(δ) including the information for a marker gene selectable in eucaryotic cells;
into an eukaryotic cell;
(b) establishing a cell line containing the Herpes helper virus vector DNA in stable form;
(c) introduction of a gene vector DNA to be packaged having at least
(α) a cis-acting signal sequence for packaging of the gene vector DNA into a viral particle of the Herpes helper virus;
(β) a gene of interest;
into the eukaryotic cell.
(d) induction of the lytic phase of the Herpes helper virus and production of the proteins important for packaging of the Herpes helper virus;
(e) packaging of the gene vector DNA into the viral particles of the Herpes helper virus; optionally
(f) releasing the viral particles containing the gene vector DNA; and/or
(g) optionally purifying the viral particles.

2. Method according to claim 1,
**characterized in that** said Herpes helper virus is selected from a group comprising a genome size of ≥ 120 kbp, ≥ 150 kbp or ≥ 170 kbp.

3. Method according to claim 1 or 2, **characterized in that** the herpes virus is an α-herpes-virus, a β-herpes-virus or a γ-herpes-virus.

4. Method according to claim 3, **characterized in that** the α-herpes-virus is a herpes simplex virus (HSV) or a varicella zoster virus (VZV), the beta herpes virus is a cytomegalo-virus (CMV) and the gamma herpes virus is an Epstein-Barr virus (EBV).

5. Method according to one or more of the preceding claims,
**characterized in that** said Herpes helper virus vector DNA comprises the information for replication of the viral DNA genome in prokaryotic cells and optionally a marker gene selectable in prokaryotic cells.

6. Method according to one or more of the preceding claims,
**characterized in that** said Herpes helper virus vector DNA contains EBV DNA as helper virus DNA.

7. Method according to one or more of the preceding claims,
**characterized in that** as said cis-acting signal sequence are employed the terminal repeat sequences of EBV, the lytic origin of replication of EBV, the plasmid origin of replication, oriP, of EBV and/or the EBNA1 gene of EBV.

8. Method according to one or more of the preceding claims, **characterized in that** the marker gene is an resistance gene for antibiotics or a gene encoding protein being detectable by fluorescence.

9. Method according to one or more of the preceding claims,
**characterized in that** a human cell is used as said eukaryotic cell.

10. Method according to one or more of the preceding claims,
**characterized in that** a gene of therapeutical value is used as said gene of interest.

11. Method according to one or more of the preceding claims,
**characterized in that** into the eukaryotic cell a gene is introduced for affecting the cell tropism of the Herpes helper virus.

12. Method according to claim 11,
**characterized in that** the gene affecting the cell tropism is present on the gene vector DNA.

13. Method according to one or more of the preceding claims, **characterized in that** the mutations comprises point mutations, mutations of several nucleotides, deletions, exchanges of nucleotides and additions of nucleotides.

14. Eukaryotic helper cell for helper virus-free packaging of gene vector DNA into the virus particles of a Herpes helper virus containing at least:
(a) a Herpes helper virus vector DNA ≥ 100kbp on one or two molecules carrying a mutation designed to render one or more of the cis-acting signal sequences for packaging non-functional and having the information for the production of the virus particles which do not contain a helper virus genome, and containing the information for a marker gene selectable in eukaryotic cells;
(b) a gene vector DNA having at least
(α) a cis-acting signal sequence for packaging of the gene vector DNA into a viral particle of the helper virus;
(β) a gene of interest.

15. Eukaryotic helper cell according to claim 14,
**characterized in that** the Herpes helper virus vector DNA contains EBV DNA as helper virus DNA.

16. Eukaryotic helper cell according to claim 14 or 15,
**characterized in that** it is a human cell.

17. Viral particles or virus DNA which may be obtained by a method according to one or more of the preceding claims.

## Revendications

1. Procédé pour l'encapsidation sans virus auxiliaire d'un ADN vecteur de gène dans les particules virales d'un herpèsvirus, comportant les étapes suivantes :
(a) introduction d'un ADN vecteur d'herpèsvirus auxiliaire sur une ou deux molécules présentant au moins les caractéristiques suivantes :
(α) une taille totale ≥100 kpb ;
(β) comporte au moins une mutation qui est conçue de telle façon qu'une ou plusieurs des séquences signal agissant en *cis* ne sont pas fonctionnelles dans l'encapsidation de l'ADN vecteur de virus auxiliaire ;
(y) comporte l'information requise pour la production des particules virales de l'herpèsvirus auxiliaire qui ne contiennent pas de génome de virus auxiliaire ;
(δ) comporte l'information pour un gène marqueur sélectionnable dans des cellules eucaryotes ;
dans une cellule eucaryote ;
(b) établissement d'une lignée cellulaire qui contient sous forme stable l'ADN vecteur d'herpèsvirus auxiliaire ;
(c) introduction d'un ADN vecteur de gène à encapsider, comportant au moins
(α) une séquence signal agissant en *cis* pour l'encapsidation de l'ADN vecteur de gène dans une particule virale de l'herpèsvirus auxiliaire ;
(β) un gène d'intérêt,
dans la cellule eucaryote ;
(d) introduction de la phase lytique de l'herpèsvirus auxiliaire et production des protéines de l'herpèsvirus auxiliaire, importantes pour l'encapsidation ;
(e) encapsidation de l'ADN vecteur de gène dans les particules virales de l'herpèsvirus auxiliaire ; éventuellement
(f) libération de particules virales contenant de l'ADN vecteur de gène ; et/ou
(g) éventuellement purification des particules virales.

2. Procédé selon la revendication 1, **caractérisé en ce que** l'herpèsvirus auxiliaire est choisi dans un groupe comprenant une taille de génome de ≥120 kpb, ≥150 kpb ou ≥170 kpb.

3. Procédé selon la revendication 1 ou 2, **caractérisé en ce que** l'herpès virus est un herpèsvirus alpha, un herpèsvirus bêta ou un herpèsvirus gamma.

4. Procédé selon la revendication 3, **caractérisé en ce que** l'herpèsvirus alpha est un virus Herpès simplex (HSV) ou un virus de varicelle-zona (VCZ), l'herpèsvirus bêta est un cytomégalovirus (CMV) et l'herpèsvirus gamma est un virus d'Epstein-Barr (EBV).

5. Procédé selon une ou plusieurs des revendications précédentes, **caractérisé en ce que** l'ADN vecteur d'herpèsvirus auxiliaire comprend l'information pour la réplication du génome d'ADN viral dans des cellules procaryotes et éventuellement un gène marqueur sélectionnable dans des cellules procaryotes.

6. Procédé selon une ou plusieurs des revendications précédentes, **caractérisé en ce que** l'ADN vecteur d'herpèsvirus auxiliaire contient de l'ADN d'EBV en tant qu'ADN de virus auxiliaire.

7. Procédé selon une ou plusieurs des revendications précédentes, **caractérisé en ce qu'**on utilise en tant que séquence signal agissant en *cis* les séquences répétées terminales de l'EBV, l'origine de réplication lytique de l'EBV, l'origine de réplication plasmidique oriP de l'EBV et/ou le gène EBNA1 de l'EBV.

8. Procédé selon une ou plusieurs des revendications précédentes, **caractérisé en ce que** le gène marqueur est un gène de résistance à un antibiotique ou un gène qui code pour une protéine détectable par fluorescence.

9. Procédé selon une ou plusieurs des revendications précédentes, **caractérisé en ce qu'**on utilise comme cellule eucaryote une cellule humaine.

10. Procédé selon une ou plusieurs des revendications précédentes, **caractérisé en ce qu'**on utilise comme gène d'intérêt un gène d'intérêt thérapeutique.

11. Procédé selon une ou plusieurs des revendications précédentes, **caractérisé en ce qu'**on introduit un gène dans la cellule eucaryote pour influer sur le tropisme cellulaire de l'herpèsvirus auxiliaire.

12. Procédé selon une la revendication 11, **caractérisé en ce que** 1e gène influant sur le tropisme cellulaire est présent sur l'ADN vecteur de gène.

13. Procédé selon une ou plusieurs des revendications précédentes, **caractérisé en ce que** la mutation comprend des mutations ponctuelles, des mutations concernant plusieurs nucléotides, des délétions, des échanges de nucléotides et des additions.

14. Cellule auxiliaire eucaryote pour l'encapsidation sans virus auxiliaire d'ADN vecteur de gène dans les particules virales d'un herpèsvirus auxiliaire, contenant au moins :
(a) un ADN vecteur d'herpèsvirus auxiliaire de ≥100 kpb, sur une ou deux molécules, comportant une mutation qui est conçue de telle façon qu'une ou plusieurs des séquences signal agissant en *cis* ne sont pas fonctionnelles dans l'encapsidation, avec l'information pour la production des particules virales qui ne contiennent pas de génome de virus auxiliaire et avec l'information pour un gène marqueur sélectionnable dans des cellules eucaryotes,
(b) un ADN vecteur de gène comportant au moins
(α) une séquence signal agissant en *cis* pour l'encapsidation de l'ADN vecteur de gène dans une particule virale du virus auxiliaire ;
(β) un gène d'intérêt.

15. Cellule auxiliaire eucaryote selon la revendication 14, **caractérisée en ce que** l'ADN vecteur d'herpèsvirus auxiliaire contient, en tant qu'ADN de virus auxiliaire, de l'ADN d'EBV.

16. Cellule auxiliaire eucaryote selon la revendication 14 ou 15, **caractérisée en ce qu'**elle est une cellule humaine.

17. Particule virale ou ADN viral, susceptible d'être obtenus par un procédé selon une ou plusieurs des revendications précédentes.
